# EUROPEAN PATENT APPLICATION

(11) **EP 4 023 231 A1**
(43) Date of publication of application: **06.07.2022**
(21) Application number: 20383170.6
(22) Date of filing: 29.12.2020
(51) Int. Cl.: A61K 35/28, C07K 14/47, C12N 9/12, C12N 5/0775

(54) **EXTRACELLULAR VESICLES DERIVED FROM MESENCHYMAL STROMAL CELLS GENETICALLY MODIFIED TO OVEREXPRESS HIF-1A AND HTERT**

(71) Applicant: Fundación Para la Investigación del Hospital Universitario y Politécnico La Fe de la Comunidad Valenciana, 46026 Valencia (ES)
(72) Inventor: DORRONSORO GONZÁLEZ, Akaitz, 46026 Valencia (ES); GÓMEZ FERRER, Marta, 46026 Valencia (ES); SEPÚLVEDA SANCHIS, Pilar, 46026 Valencia (ES)
(74) Representative: Herrero & Asociados, S.L.

(57) **Abstract**

The present invention refers to extracellular vesicles (EVs) isolated from mesenchymal stromal cells genetically modified to overexpress HIF-1α and hTERT (MSCs-T-HIF). The present invention also refers to the method for obtaining the extracellular vesicles. Finally, the clinical applications of the EVs in cellular therapy, in particular for the treatment of autoimmune diseases and ischemic diseases, are also contemplated.

SEQUENCE LISTING

<110>  FUNDACIÓN PARA LA INVESTIGACIÓN DEL HOSPITAL UNIVERSITARIO
       Y POLITÉCNICO LA FE DE LA COMUNIDAD VALENCIANA

<120>  Extracellular vesicles derived from mesenchymal stromal cells
       genetically modified to overexpress HIF-1 alpha and hTERT

<130>  225/2020

<160>  16

<170>  PatentIn version 3.5

<210>  1
<211>  18
<212>  DNA
<213>  Artificial Sequence

<220>
<223>  TS PRIMER

<400>  1
aatccgtcga gcagagtt                                                    18


<210>  2
<211>  30
<212>  DNA
<213>  Artificial Sequence

<220>
<223>  ACX Reverse Primer

<400>  2
gcgcggctta cccttaccct taccctaacc                                       30


<210>  3
<211>  19
<212>  DNA
<213>  Artificial Sequence

<220>
<223>  GAPDH-Human specific sense primer

<400>  3
cccctctgct gatgcccca                                                   19


<210>  4
<211>  20
<212>  DNA
<213>  Artificial Sequence

<220>
<223>  GAPDH-Human specific antisense primer

<400>  4
tgaccttggc caggggtgct                                                  20


<210>  5

<211>  20
<212>  DNA
<213>  Artificial Sequence

<220>
<223>  TERT-human specific sense primer

<400>  5
gtatggctgc gtggtgaact                                                    20


<210>  6
<211>  20
<212>  DNA
<213>  Artificial Sequence

<220>
<223>  TERT-Human specific antisense primer

<400>  6
tctgaacaaa agccgtgcca                                                    20


<210>  7
<211>  19
<212>  DNA
<213>  Artificial Sequence

<220>
<223>  IDO-Human specific sense primer

<400>  7
ctaccatctg caaatcgtg                                                     19


<210>  8
<211>  19
<212>  DNA
<213>  Artificial Sequence

<220>
<223>  IDO_Human specific antisense primer

<400>  8
gaagggtctt cagaggtct                                                     19


<210>  9
<211>  19
<212>  DNA
<213>  Artificial Sequence

<220>
<223>  COX-2_Human specific sense primer

<400>  9
gaatcattca ccaggcaaa                                                     19


<210>  10
<211>  20
<212>  DNA
<213>  Artificial Sequence

<220>
<223>  COX-2_Human specific antisense primer

<400>  10
tctgtactgc gggtggaaca                                                   20


<210>  11
<211>  20
<212>  DNA
<213>  Artificial Sequence

<220>
<223>  PD-L1_Human specific sense primer

<400>  11
cctgcagggc attccagaaa                                                   20


<210>  12
<211>  20
<212>  DNA
<213>  Artificial Sequence

<220>
<223>  PD-L1_Human specific antisense primer

<400>  12
gtccttggga accgtgacag                                                   20


<210>  13
<211>  20
<212>  DNA
<213>  Artificial Sequence

<220>
<223>  IL-6_Human specific sense primer

<400>  13
ccaggagccc agctatgaac                                                   20


<210>  14
<211>  18
<212>  DNA
<213>  Artificial Sequence

<220>
<223>  IL-6_Human specific sense primer

<400>  14
gagcagcccc agggagaa                                                     18


<210>  15
<211>  17
<212>  DNA
<213>  Artificial Sequence

<220>
<223>  HIF-1 alpha_Human specific sense primer

<400>  15
cacagcctgg atatgaa                                                    17

<210>  16
<211>  21
<212>  DNA
<213>  Artificial Sequence

<220>
<223>  HIF-1 alpha_Human specific antisense primer

<400>  16
gaattcttgg tgttatatat g                                               21

2

## Description

### Field of the invention

The present invention relates generally to the Health Sector, in particular the field of Immunology and Tissue Regeneration. Particularly, the invention relates to Extracellular Vesicles (EVs) derived from mesenchymal stromal cells genetically modified to overexpress HIF-1α and hTERT (MSCs-T-HIF). The invention also contemplates the method for obtaining said EVs, pharmaceuticals compositions comprising said EVs, and clinical applications thereof.

### Background of the invention

Mesenchymal stromal cells (MSCs) are multipotent progenitor cells, plastic adherent in standard conditions, with a specific surface antigen expression and capable to differentiate into the chondrocyte, osteoblast or adipocyte lineages (Dominici, M. et al "Minimal criteria for defining multipotent mesenchymal stromal cells. The International Society for Cellular Therapy position statement" Cytotherapt (2006) Vol. 8, n° 4, 315-317). During the last years, MSCs emerged as a potential therapeutic element to treat multiple diseases based on both their tissue regeneration and immunosuppressive capacity (Dorronsoro, A. et al. Human mesenchymal stromal cell-mediated immunoregulation: mechanisms of action and clinical applications. Bone Marrow Res. 2013, 203643 (2013*).* Since 1999, multiple in vitro and preclinical studies indicated that MSCs reduce tissue damage after ischemic events in different tissues and that modulate acute and chronic inflammatory reactions (*Dorronsoro, A. et al. 2013).* Regarding the mechanism driving mentioned features, the importance of cell-to-cell contact is under discussion in the field, but several soluble factors have been confirmed to be involved in the healing trigger by stromal cells. In terms of immunosuppression, it has been shown that MSCs are able to reduce the proliferation of activated T cells in a dose dependent mainly through the expression of IDO and COX2 (Meisel, R. et al. Human bone marrow stromal cells inhibit allogeneic T-cell responses by indoleamine 2,3-dioxygenase-mediated tryptophan degradation. Blood 103, 4619-4621 (2004*);* Aggarwal, S. & Pittenger, M. F. Human mesenchymal stem cells modulate allogeneic immune cell responses. Blood 105, 1815-22 (2005*) .*

In addition, MSC favour the appearance of regulatory T cells by secreting TGF-β (English, K. et al. Cell contact, prostaglandin E2 and transforming growth factor beta 1 play non-redundant roles in human mesenchymal stem cell induction of CD4+CD25Highforkhead box P3+ regulatory T cells. Clin. Exp. Immunol. 156, 149-160 (2009*).* At the same time, MSC are also able to inhibit the activation of M1 macrophages and promote M1 to M2 polarization (Németh, K. et al. Bone marrow stromal cells attenuate sepsis via prostaglandin E 2-dependent reprogramming of host macrophages to increase their interleukin-10 production. Nat. Med. 15, 42-49 (2009*);* Abumaree, M. H. et al. Human Placental Mesenchymal Stem Cells (pMSCs) Play a Role as Immune Suppressive Cells by Shifting Macrophage Differentiation from Inflammatory M1 to Anti-inflammatory M2 Macrophages. Stem Cell Rev. Reports 9, 620-641 (2013*).*

Regarding NK cells, MSC reduce the proliferation and cytotoxic activity of these cells (Aggarwal, S. et al 2005; Rasmusson, I., Ringdén, O., Sundberg, B. & Le Blanc, K. Mesenchymal stem cells inhibit the formation of cytotoxic T lymphocytes, but not activated cytotoxic T lymphocytes or natural killer cells. Transplantation 76, 1208-13 (2003*);* Spaggiari, G. M., Capobianco, A., Becchetti, S., Mingari, M. C. & Moretta, L. Mesenchymal stem cell-natural killer cell interactions: Evidence that activated NK cells are capable of killing MSCs, whereas MSCs can inhibit IL-2-induced NK-cell proliferation. Blood 107, 1484-1490 (2006*).* These properties, in combination with the fact that MSC preferentially home to damaged tissue, made them good candidates for cell-based therapeutic strategies to treat a wide range of disorders. However, until date, most of the clinical trials performed had been inconsistent and did not show unequivocal efficacy. So far, only the trial performed by Tigenix in 2018, for the treatment of refractory rectovaginal anal fistula in the setting of Crohn's disease, achieved undoubtfully positive results. Nonetheless, no study reported adverse events directly related to MSC infusion what supports further studies aiming to develop a therapeutic strategy than could efficiently be translated from bench to bed-side (Galipeau, J. & Sensébé, L. Mesenchymal Stromal Cells: Clinical Challenges and Therapeutic Opportunities. Cell Stem Cell 22, 824-833 (2018*).*

Small extracelullar vesicles (EVs) are small particles 30 to 200 nm in diameter and delimited by a lipidic bilayer membrane. EVs contain a diverse cargo composed by different cytokines, membrane trafficking molecules, chemokines, heat shock proteins and even mRNA and microRNAs (Théry, C. et al. Minimal information for studies of extracellular vesicles 2018 (MISEV2018): a position statement of the International Society for Extracellular Vesicles and update of the MISEV2014 guidelines. J. Extracell. Vesicles 7, 1535750 (2018*);* Théry, C., Zitvogel, L. & Amigorena, S. Exosomes: Composition, biogenesis and function. Nature Reviews Immunology 2, 569-579 (2002*).*

Vesicle-based therapeutic strategies have great advantages in terms of biosafety and production under clinical-grade conditions. Since they are not living elements and do not carry nuclear DNA, EVs transplantation involves a lower risk of causing the growth of foreign bodies or malignant tumors. Similarly, the use of EVs facilitates pharmaceutical production and clinical logistics, significantly reducing the cost of a potential therapy.

Multiple preclinical studies showed that EVs secreted by MSC are able to recapitulate different therapeutic results previously observed by using MSCs. Among these studies, different authors showed that EVs secreted by MSCs modulated the activity of different immune cells both in vitro an in vivo (Phinney, D. G. & Pittenger, M. F. Concise Review: MSC-Derived Exosomes for Cell-Free Therapy. Stem Cells 35, 851-858 (2017*).* Moreover, EVs secreted by MSCs migrate to damage tissues after in vivo transplantation since, EVs membrane is highly similar to the membrane of the secreting cells and provides them with similar homing and engraftment features.

HIF-1α is a master regulator of adaptation to hypoxia (Ratcliffe, P. J. From erythropoietin to oxygen: Hypoxia-inducible factor hydroxylases and the hypoxia signal pathway. in Blood Purification 20, 445-450 (2002*)* that modulates different cellular processes in MSCs such as survival, proliferation, migration and differentiation (Stubbs, S. L. et al. Hypoxic preconditioning enhances survival of human adipose-derived stem cells and conditions endothelial cells in vitro. Stem Cells Dev. 21, 1887-1896 (2012*)).* Several reports have demonstrated significant improvements on the regeneration potential of MSCs after hypoxia preconditioning (Zou, D. et al. Repair of critical-sized rat calvarial defects using genetically engineered bone marrow-derived mesenchymal stem cells overexpressing hypoxia-inducible factor-1α. Stem Cells 29, 1380-1390 (2011*);* Cerrada, I. et al. Hypoxia-Inducible Factor 1 Alpha Contributes to Cardiac Healing in Mesenchymal Stem Cells-Mediated Cardiac Repair. Stem Cells Dev. 22, 501-511 (2013*).*

In close relation, it has been previously demonstrated that overexpression of HIF-1α potentiates immunosuppressive capacity of MSCs over immune cell populations such as dendritic cells, NK cells and monocytes (Martinez, V. G. et al. Overexpression of hypoxia-inducible factor 1 alpha improves immunomodulation by dental mesenchymal stem cells. Stem Cell Res. Ther. 8, 208 (2017*).* Besides, the immunosuppressive ability of MSCs is not constitutive but rather induced by crosstalk with different cells of the immune system. This extent is relevant for any EVs based therapy since their healing features depend on physiological state of parental cells at the moment of secretion. In this regard, immunosuppressive properties of MSC must be activated by external stimuli in order to transfer that capacity to EVs before they are collected (Joo, Suh, Lee, Bang & Lee. Current Knowledge and Future Perspectives on Mesenchymal Stem Cell-Derived Exosomes as a New Therapeutic Agent. Int. J. Mol. Sci. 21, 727 (2020*).*

Currently, there are different pathologies related to the unwanted or uncontrolled activation of the immune system for which there is no effective solution, such as graft-versus-host disease, acute rejection of a transplanted organ or autoimmune pathologies.

Mesenchymal stromal cells and the vesicles secreted by them have generated promising results in different animal models, but when these results were transferred to clinical trials, the result has not reached the desired expectations. The trials carried out so far show that the potency of the effect is limited and therefore the dose required to obtain results is very high. At the same time, MSCs do not proliferate in an unlimited manner and, after a short period of time, increase their senescence. Therefore, any MSC- or EV-secreted therapy based on MSC requires changing donors with some frequency, which seriously hinders standardization between batches and makes the process significantly more expensive.

The authors of the present invention, after a significant research effort, have developed a new strategy to improve therapeutic capacity of EVs combining genetic modifications and proinflammatory stimulation on MSCs to obtain a constant source of boosted immunosuppressive EVs. In particular, they have generated a genetically modified MSC cell line, by overexpressing Hypoxia inducible factor 1 α (HIF-1α) and human telomerase (hTERT), to be used as EVs source. Secreted EVs have an improved therapeutic capacity which is further increased by a specific cytokine based preconditioning culture medium.

### Brief description of the drawings

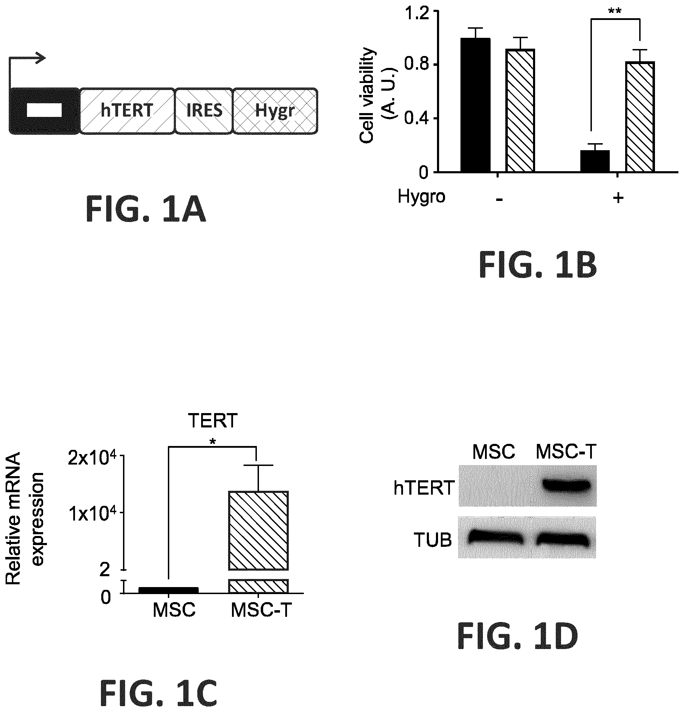

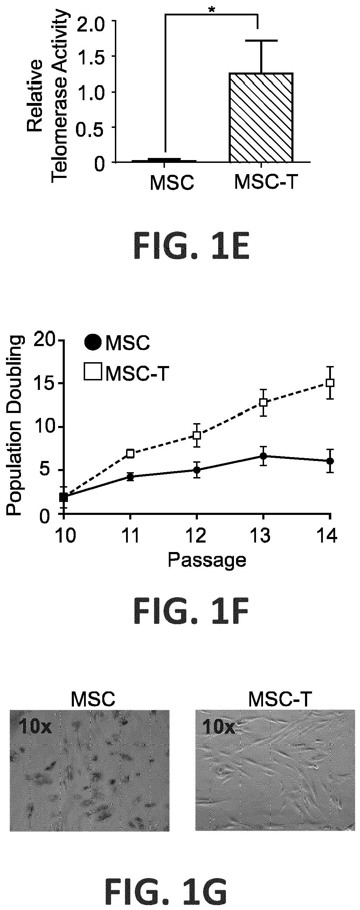

**Figure 1****.** Generation and validation of stable and non- senescence cell line. A) pLV-hTERT-Hygromyn lentiviral vector. B) Quantification of viable MSC (black) and MSC-T (grey) detected by MTT assay treated or not with hygromycin for 48h. Values are represented as relative to MSC not treated condition and graphs represent the mean and SD of 3 independent experiments C) hTERT gene expression levels quantified by RT-qPCR in MSCs (black) and MSCs-T (grey). The expression levels of the target gene in each sample have been normalized to GAPDH expression. Values are represented as relative to MSCs levels. Graphs represent mean and SD of 3 independent experiments. D) Representative picture of hTERT protein detected by western blotting; α-tubulin was used as loading control. E) Relative telomerase activity (RTA) by relative-quantitative telomerase repeat amplification protocol assay (RQ-TRAP) in 1x106 MSC (black) and MSC-T (grey). Graphs represent the mean and SD of 3 independent experiments. F) Proliferation assay. MSC (black) and MSC-T (red) growth curve; represented by doubling time at different passages during cell culture. Each point represents the mean and SD of 2 independent experiments G) Representative picture of senescence associated β-Galactosidase detection on MSC (left) and MSC-T (right) at passage 14. *p < 0.05, ***p < 0.001

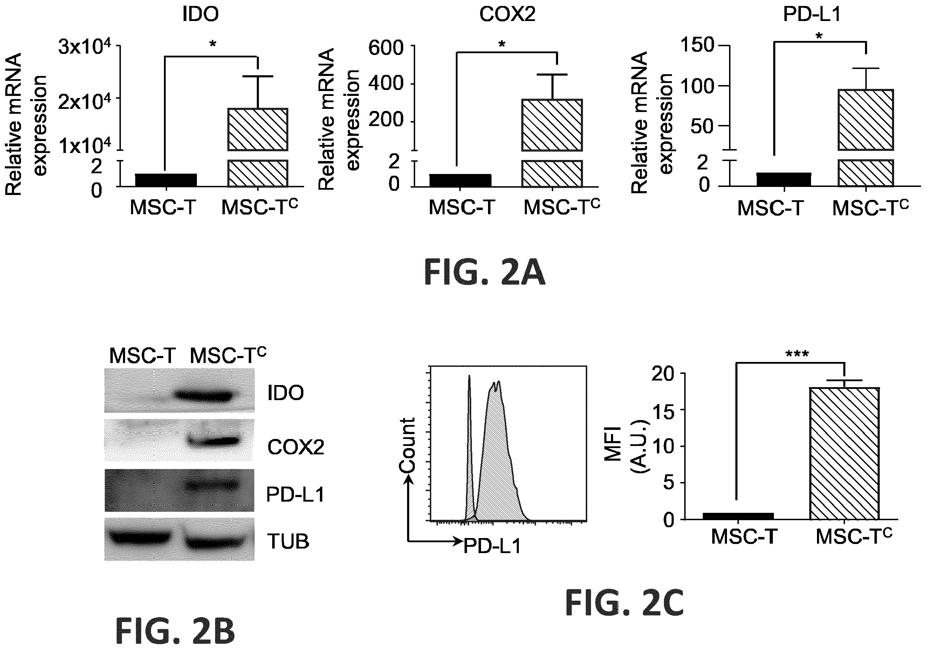

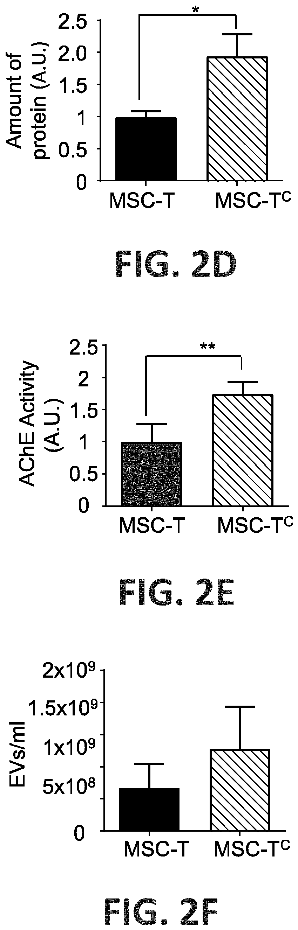

**Figure 2****.** Conditioning medium enriches immunomodulatory capacity of cells. Quantification of immunosuppressive molecules and amount of EVs released by MSCs conditioned (MSC-T^{c}) or not (MSC-T) with pro-inflammatory cytokines during 48h. A) IDO, COX2 and PD-L1 gene expression levels measured by RT-qPCR in MSCs-T (black) and MSC-T^{c} (grey). The expression levels of the target gene in each sample have been normalized to GAPDH expression. Values are represented as relative to MSCs levels. Graphs represent mean and SD of 3 independent experiments. B) Representative picture of IDO, COX2 and PD-L1 protein expression levels detected by WB, α-tubulin was used as a protein loading control. C) PD-L1 protein expression level measured by flow cytometry using geometric mean value of MSC-T (black) and MSC-T^{c} (grey). Values are represented as relative to MSC-T geometric mean value. Graphs represent mean and SD of 3 independent experiments. D) Concentration of total protein determined with BCA kit of EVs extracted from same cell number of MSC-T (black) and MSC-Tc (grey). Values are represented as relative to MSCs-T value. Graphs represent mean and SD of 4 independent experiments E) Quantification of acetylcholinesterase activity in EVs of MSC-T (EV_{MSC-T}) and MSC-T^{c} (EV_{MSC-T}^{c}). Values are represented as relative to EV_{MSC-T} value. Graph represent mean and SD of 3 independent experiments. F) Amount of EVs per ml in the supernatant of same number of MSC-T (black) and MSC-T^{c} (grey) measure by NTA. Graphs represent mean and SD of 3 independent experiments. *p < 0.05, **p < 0.01; t-test assuming equal variance.

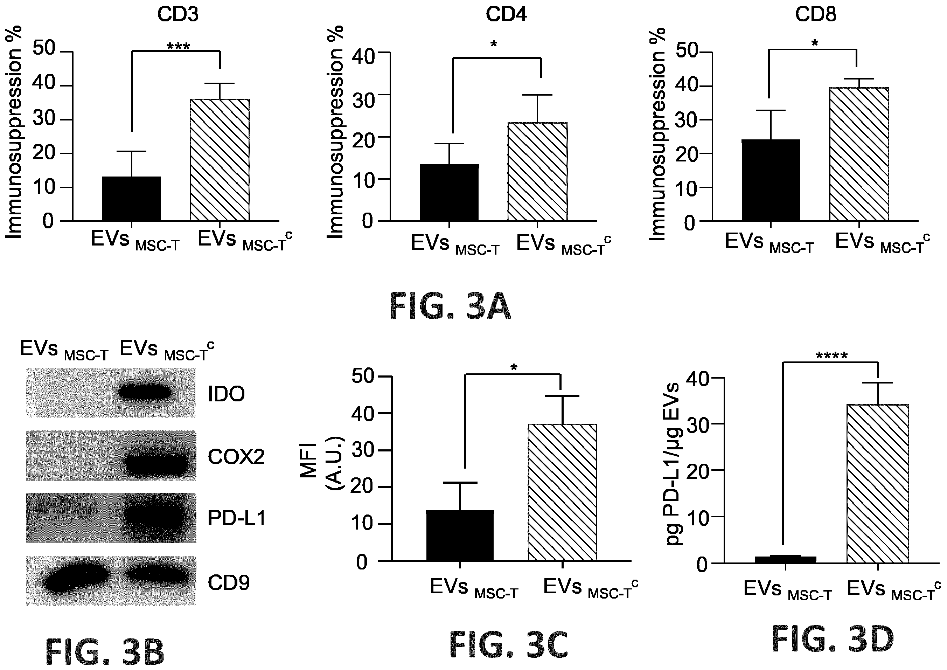

**Figure 3****.** Conditioning results in an enrichment of immunomodulatory capacity of EV_{MSC-T}. A) PBMCs were stained with CFSE and stimulated with anti-CD3 and anti-CD28 beads in presence or absence of EV_{MSC-T} (black) and EV_{MSC-T}^{c}(grey). After 5 days, cells were stained with α-CD3, α-CD4, and α-CD8 antibodies and proliferation of T-cell subsets was determined by flow cytometry measuring CFSE dilution. Suppression percentage was calculated based on expansion index. Graph represent mean and SD of 4 independent experiments. B) Representative picture of IDO, COX2 and PD-L1 protein expression levels detected by WB. CD9 was used as a loading control. C) PD-L1 protein expression level measured by flow cytometry using geometric mean value of EV_{MSC-T} (black) and EV_{MSC-T}^{c}(grey). Values are represented as relative to EV_{MSC-T} geometric mean value. Graphs represent mean and SD of 3 independent experiments. D) PD-L1 protein expression levels measured by ELISA in EV_{MSC-T} (grey) and EV_{MSC-T}^{c}(black). Graphs represent mean and SD of 3 independent experiments. *p < 0.05, ***p < 0.001

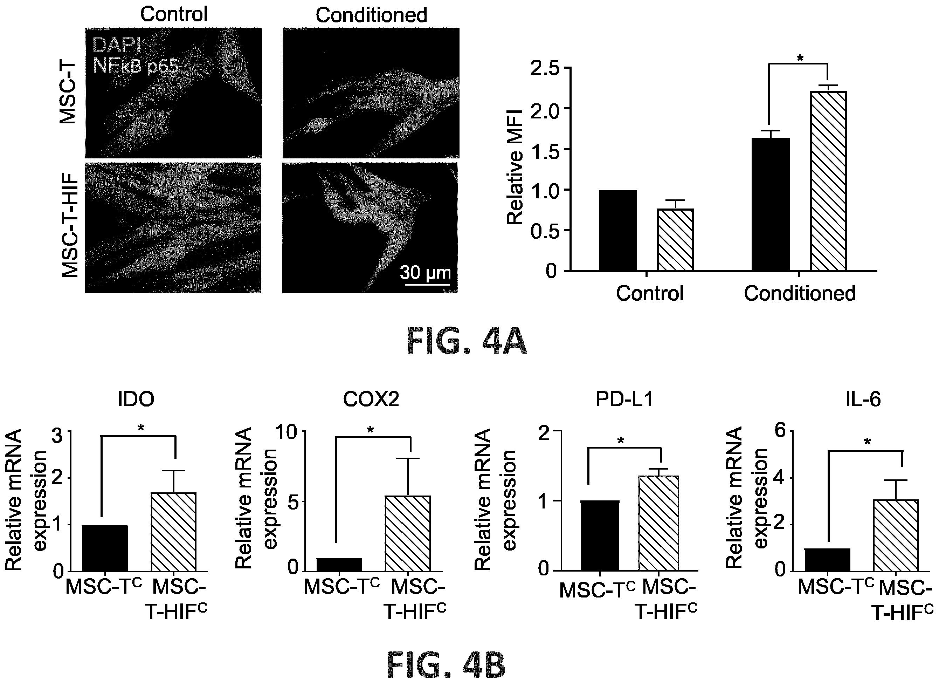

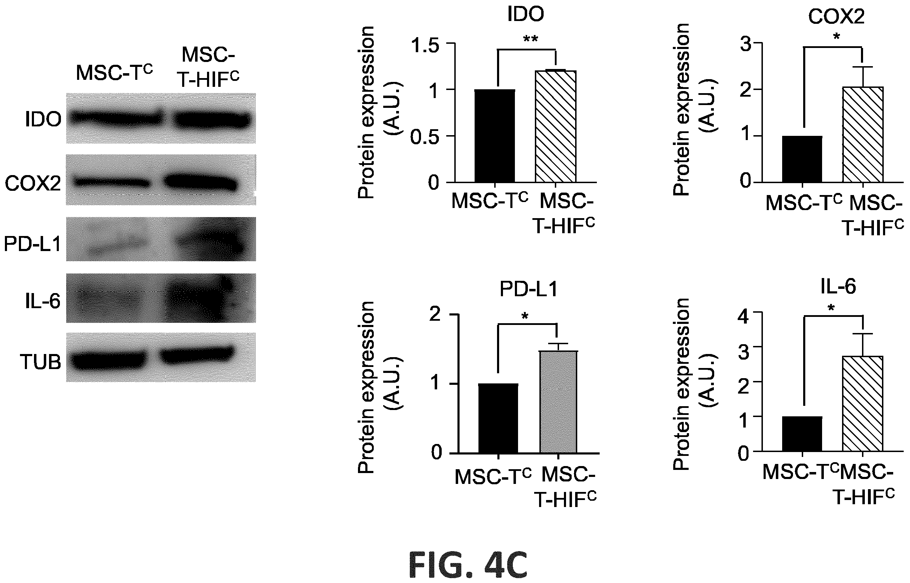

**Figure 4****.** HIF overexpression increased signalling through NF-κB pathway and consequently the expression of immunosuppressive cytokines. A) Immunolocalization of p65 in MSC-T, MSC-T^{c}, MSC-T-HIF and MSC-T-HIF^{c}. Red: p65, Blue: DAPI. Scale bar: 30 µm. Mean of fluorescence intensity was measured per nuclei and values normalized to MSC-Ts. Graphs represent mean and SD of 75 cells. B) IL-6, IDO and COX2 gene expression levels quantified by RT-qPCR in MSC-T^{c}s (black) and MSC-T-HIF^{c}s (grey). The expression levels of the target gene in each sample have been normalized to GAPDH expression. Graph represent mean and SD of fold change of 3 independent experiments. C) Representative picture of IL-6, IDO and COX2 protein detected by WB. Expression levels were quantified by densitometry relative to MSC-T^{c}. α-tubulin was used as a protein loading control. Bars represent mean and SD of 3 independent experiments. *p < 0.05

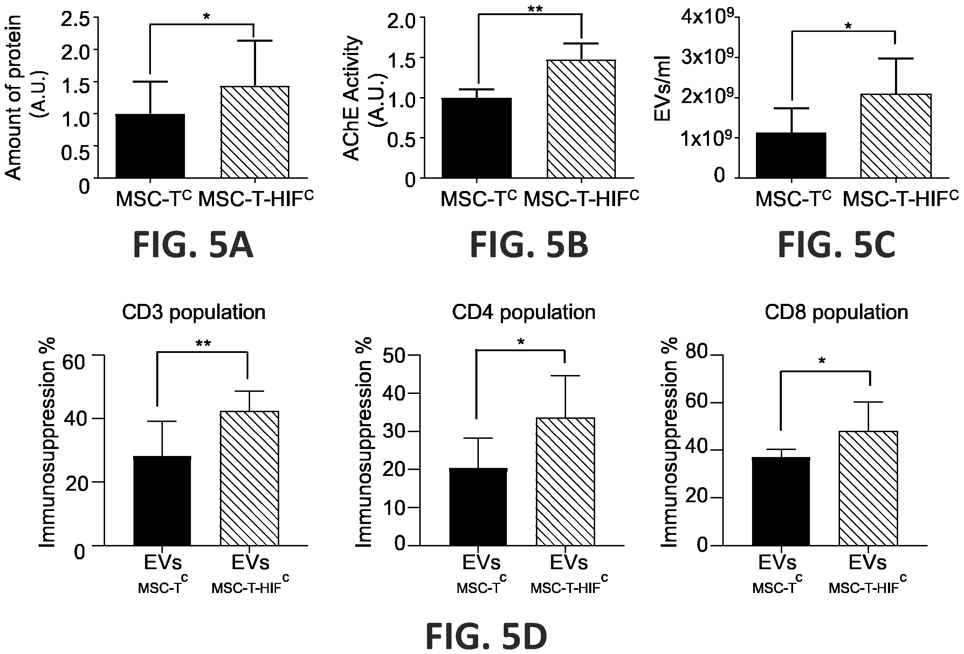

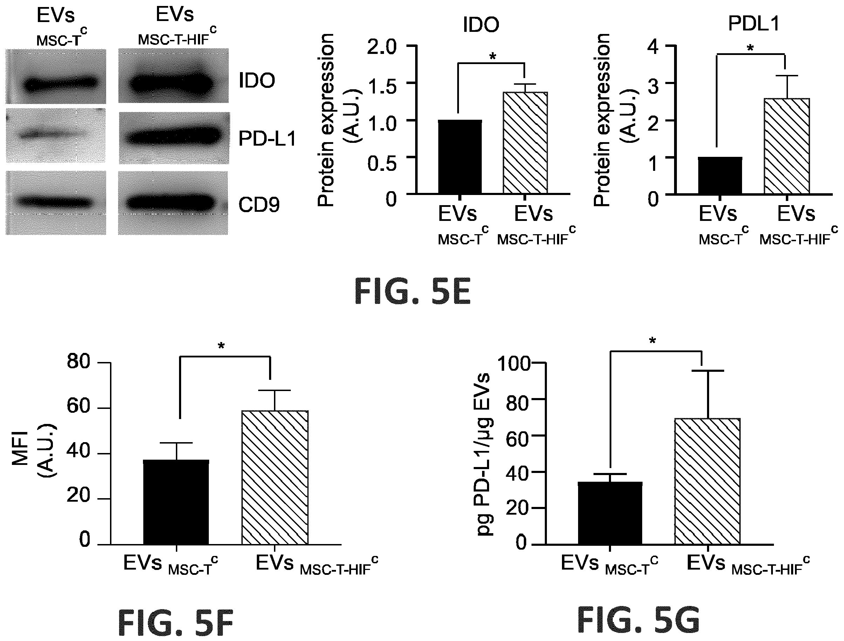

**Figure 5****.** EV_{MSC-T-HIF}^{c} display enhanced immunosuppression properties. A) Concentration of total protein determined by BCA on EV_{MSC-T}^{c} (black) and EV_{MSC-T-HIF}^{c} (grey). Values are represented as relative to EV_{MSC-T}^{c} value. Graphs represent mean and SD of 6 independent experiments. B) Quantification of acetylcholinesterase activity in EV_{MSC-T}^{c} and in EV_{MSC-T-HIF}^{c}. Values are represented as relative to EV_{MSC-T}^{c} value. Graph represent mean and SD of 3 independent experiments. C) Amount of EVs per ml in the supernatant of same number of MSC-T^{c} (black) and MSC-T-HIF^{c} (grey) measure by NTA. Graphs represent mean and SD of 3 independent experiments. D) PBMCs were stained with CFSE and stimulated with anti-CD3 and anti-CD28 beads in presence or absence of EV_{MSC-T}^{c} (black) and EV_{MSC-T-HIF}^{c} (grey). After 6 days, cells were stained with α-CD3, α-CD4, and α-CD8 antibodies and proliferation of T-cell subsets was determined by flow cytometry measuring CFSE dilution. Suppression percentage was calculated based on expansion index. Graph represent mean and SD of 7 independent experiments. E) Representative picture of IDO and PD-L1 protein detected by WB in EV_{MSC-T}^{c} and EV_{MSC-T-HIF}^{c}. Expression levels were quantified by densitometry relative to EVMSC-Tc. CD9 was used as loading control. Graph represent mean and SD of 3 independent experiments. F) PD-L1 protein expression level measured by flow cytometry using geometric mean value of EV_{MSC-T}^{c} (black) and EV_{MSC-T-HIF}^{c} (grey). Values are represented as relative to EV_{MSC-T}^{c} geometric mean value. Graphs represent mean and SD of 3 independent experiments. G) PD-L1 protein expression levels measured by ELISA in EV_{MSC-T}^{c} (grey) and EV_{MSC-T-HIF}^{c} (black). Graphs represent mean and SD of 3 independent experiments. *p < 0.05, **p < 0.01, ***p < 0.001

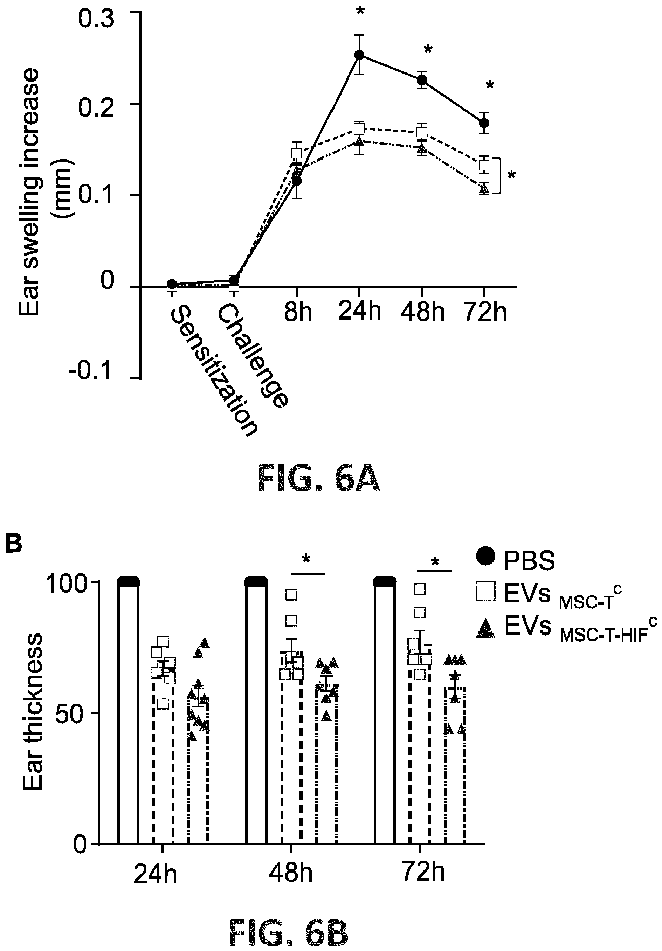

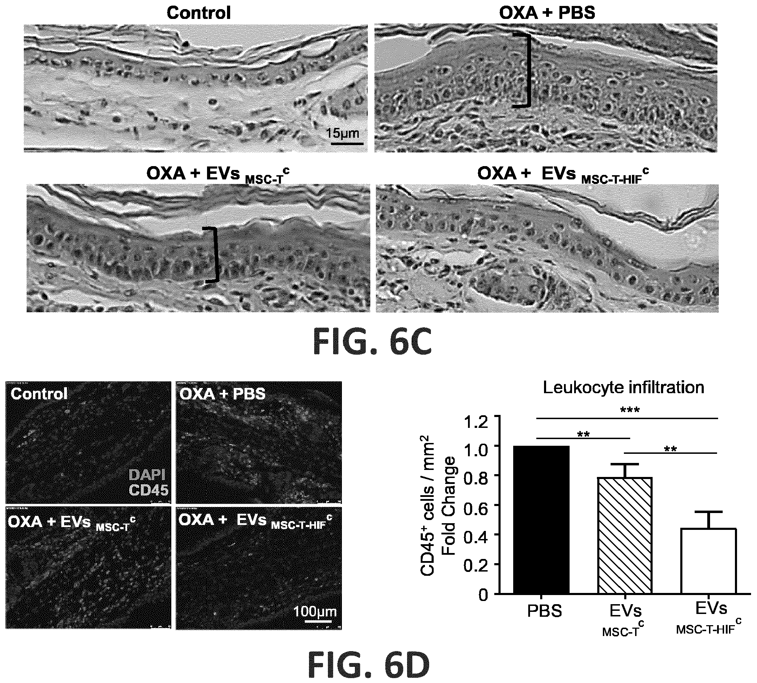

**Figure 6****.** EV_{MSC-T-HIF}^{c} show immunoregulatory potential in a Delayed-Type Hypersensitivity mouse model, reducing ear swelling and improving tissue integrity. BALB/c mice were sensitized by applying 3% oxazolone solution on the abdomen skin at day -5 and the right ear challenged with 1% oxazolone solution at day 0. Mice were injected with different treatments: PBS (black circle), EV_{MSC-T}^{c} (red triangle) or EV_{MSC-T-HIF}^{c} (blue square) 6h after challenge. Non challenged ear was used as no inflammation control. A) Ear swelling is showed as change in thickness (in mm) compared to untreated ear thickness of the same mouse at different time points. Data are presented as an average ±SEM, n=7. B) Ear thickness measured at different time points. Data is shown as reduction of thickness cause by each treatment relative to PBS injected ear. Graph represents mean and SD of 7 mice in each group. C) Representative pictures of histological analysis by H/E of ears at 72h time point. Spongiosis is pointed by arrows and hyperkeratosis is pointed by brackets. Scale bar: 100µm and 30 µm. D) Immunodetection of CD45+ cells in ear at 72h time point. Scale bar: 100µm. Quantification of CD45+ positive cells per mm2. Data is showed as relative to PBS condition. Graph represent mean of 4 mice ± SD. *p < 0.05.

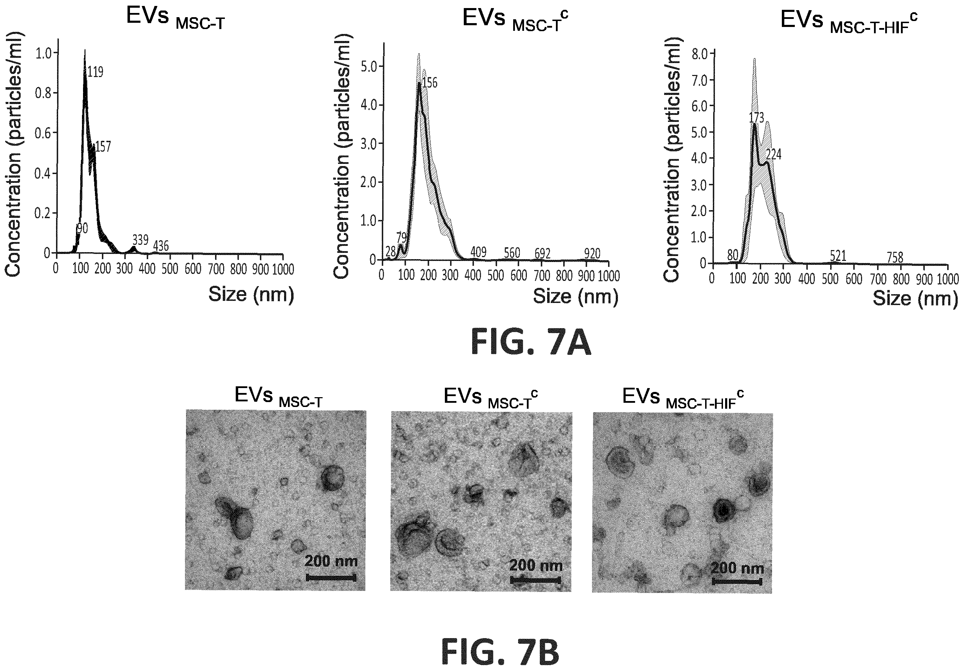

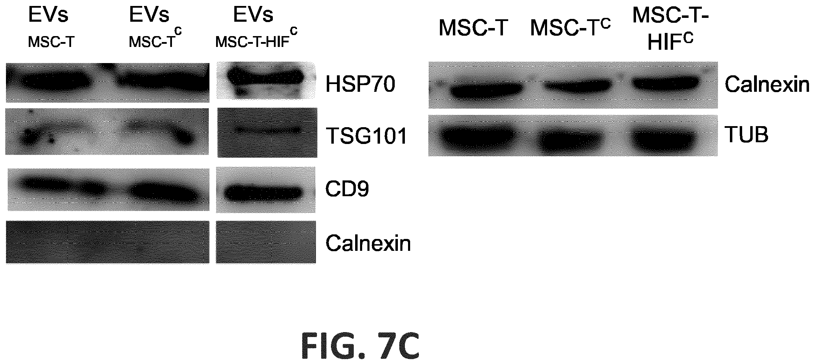

**Figure 7****.** Priming and genetic modifications do not affect inherent features of EVs such as size, morphology and expression of special markers. A) Representative images of EVs analyzed by NTA. B) Representative electron microscopy pictures of isolated EVs collected from different MSC conditions. Scale bar: 200nm. C) Representative picture of CD9, TSG101 and Hsp70 protein detection by WB in EVs. Absence of Calnexin demonstrates a pure EVs preparation. Cells were used as calnexin positive control.

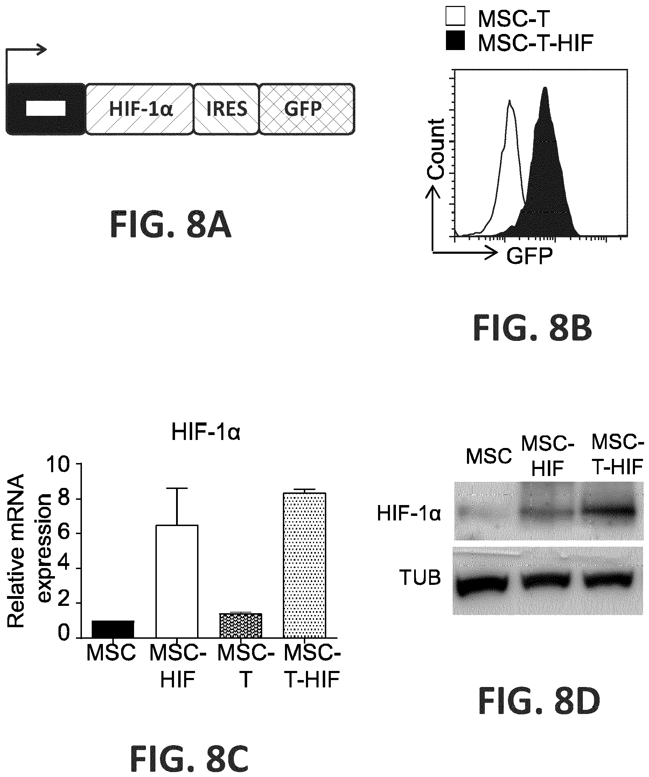

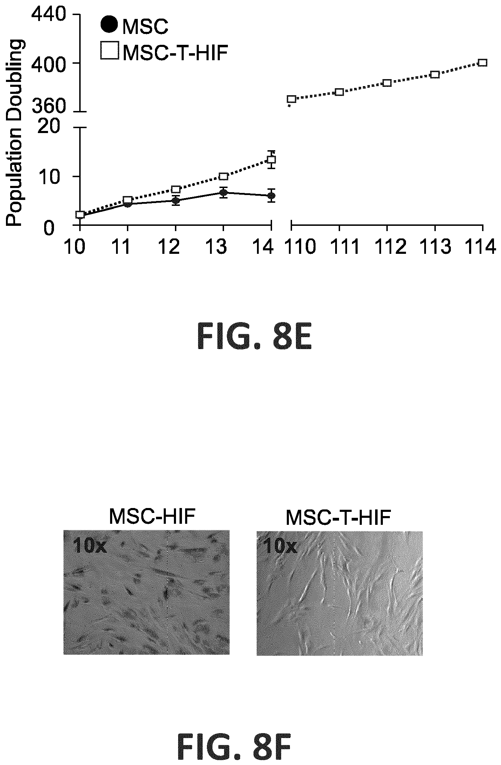

**Figure 8****.** Overexpression of HIF-1α do not affect non-senescence conditions. A) pWPI-HIF-1α-GFP lentiviral vector. B) Lentiviral transfection with pWIPI-HIF-GFP (MSCs-T-HIF) detected by flow cytometric analysis of GFP. C) HIF-1α gene expression levels detected by RT-qPCR in MSCs-HIF (white bars) and MSCs-T-HIF (stripped white bars). The expression levels of the target gene in each sample have been normalized against GAPDH expression. Graph represent mean and SD of fold change of 3 independent experiments D) Representative picture of HIF-1α protein detected by WB. α-tubulin was used as a protein loading control. E) Proliferation assay. MSC-T (red) and MSC-T-HIF (blue) growth curve; represented by doubling time at different passages during cell culture. Each point represents mean and SD of 2 independent experiments. F) Representative picture of senescence associated β-Galactosidase detection on MSC-HIF (left) and MSC-T-HIF (right) at passage 14. * p < 0.05.

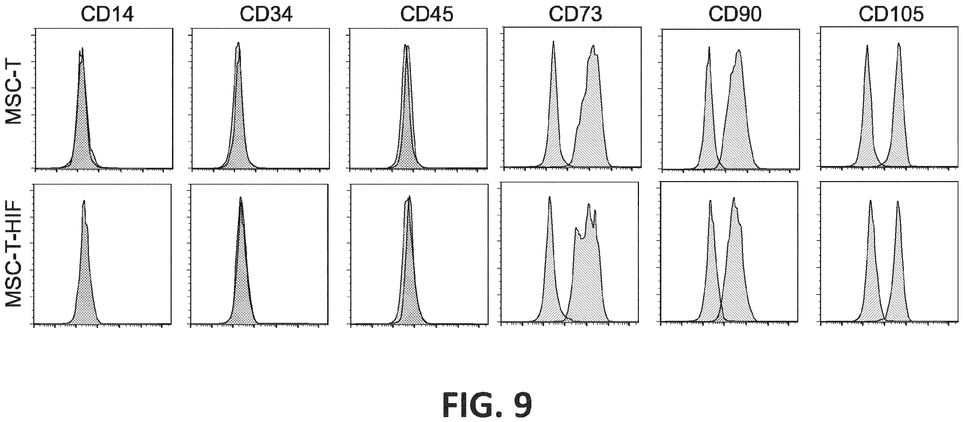

**Figure 9****.** Genetic modification does not affect the standard phenotypic features necessary to define MSCs. A) Representative picture of different MSCs lines adhere to plastic under normal culture conditions showing fibroblast-like morphology. B) Representative picture of different MSC lines differentiated towards osteoblast lineage and stained with Alizarin red. C) Representative picture of different MSC lines differentiated towards adipocyte. D) Cell surface marker profile of different MSC cell lines.

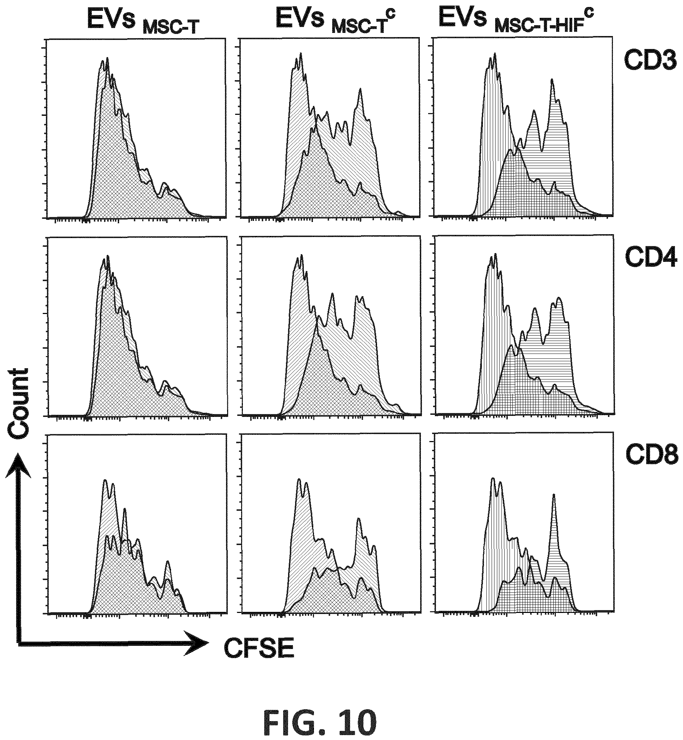

**Figure 10**. EV_{MSC-T-HIF}^{c} display enhanced immunosuppression properties. Representative flow cytometry plots of CFSE dilution in CD3+ cells activated with CD3/CD28 beads and treated with different EVs (coloured) or without EVs (grey).

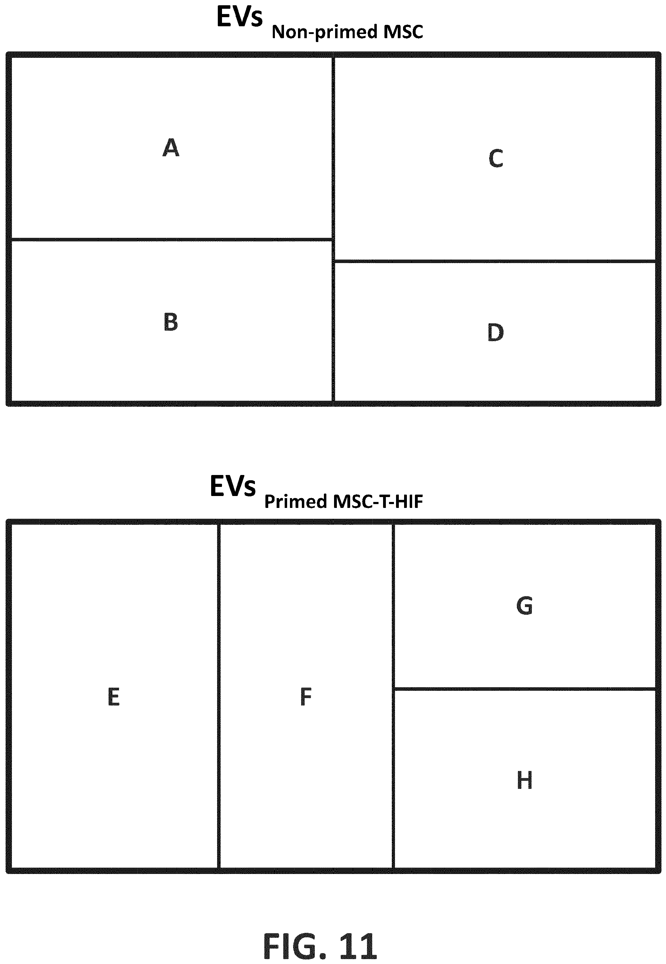

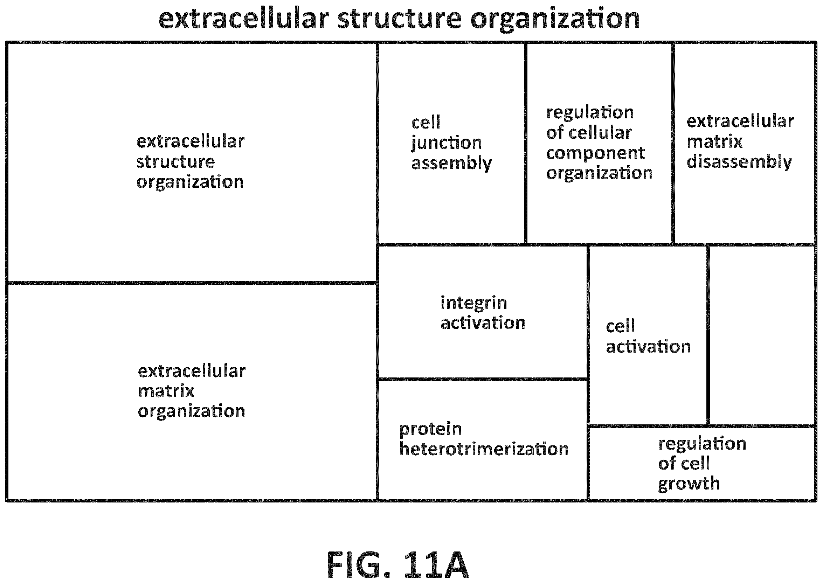

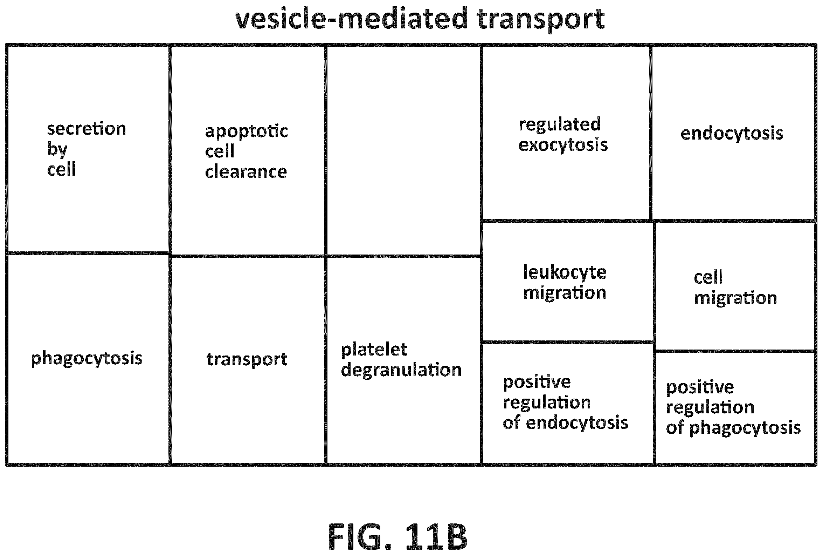

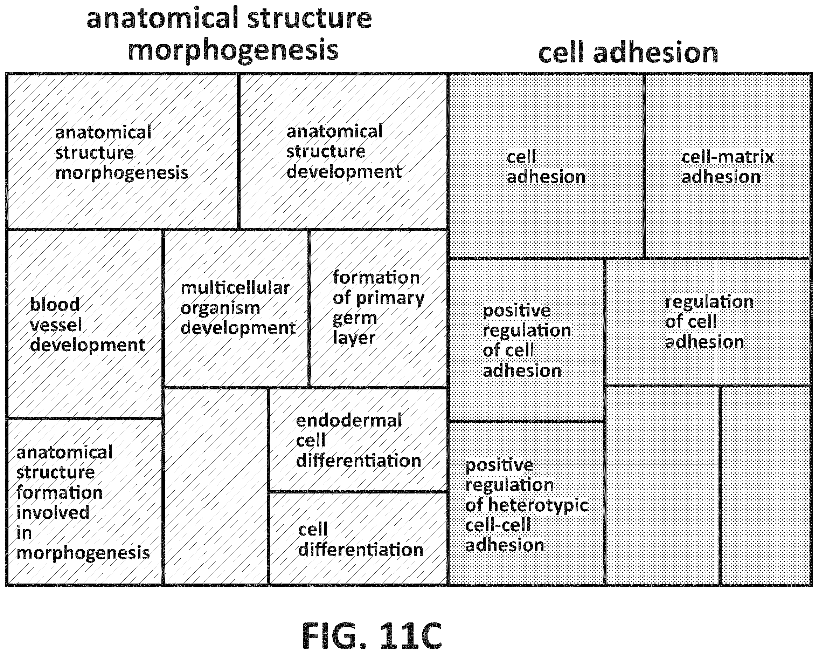

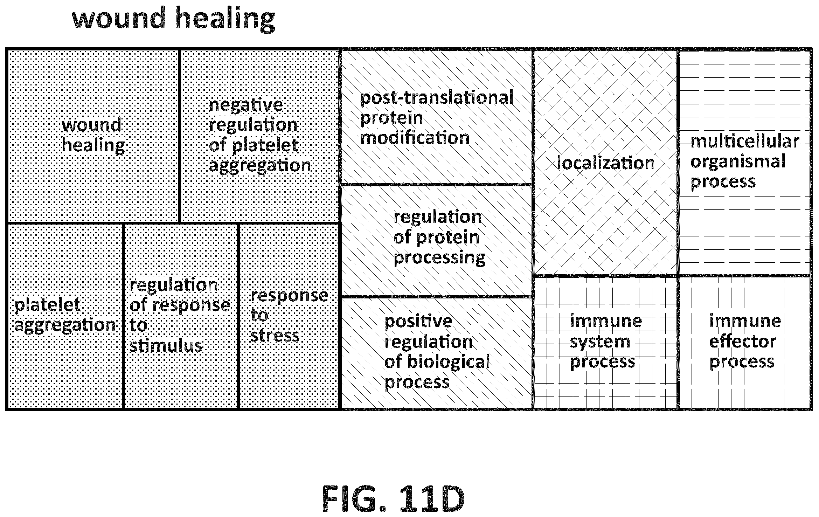

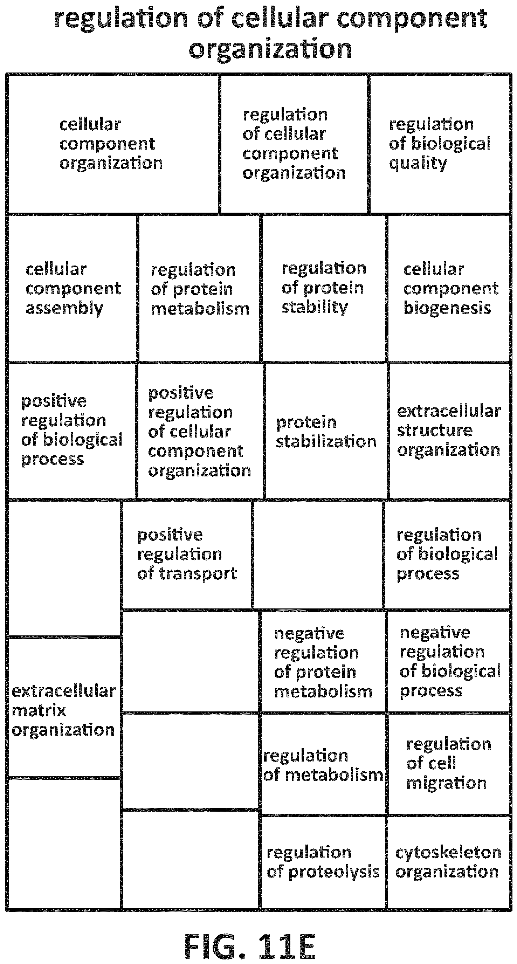

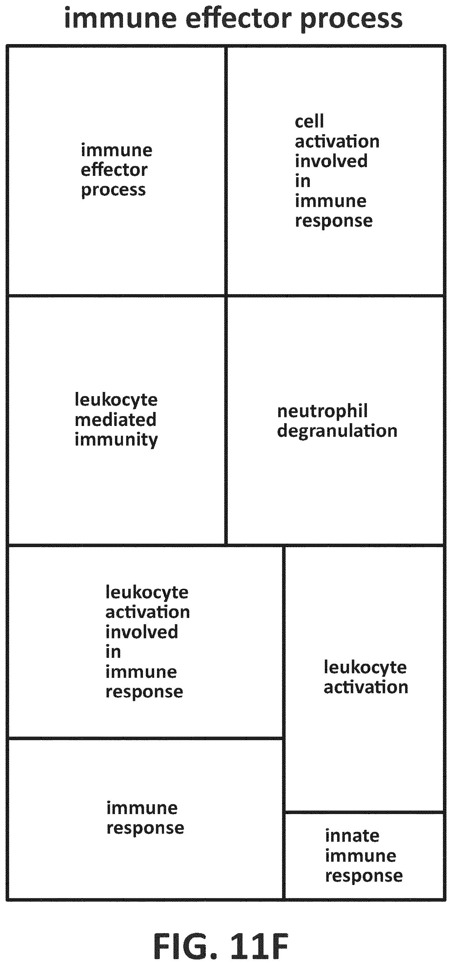

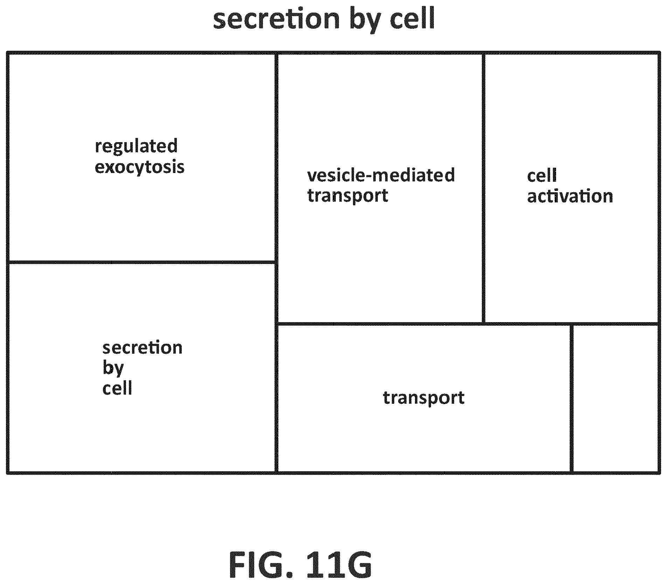

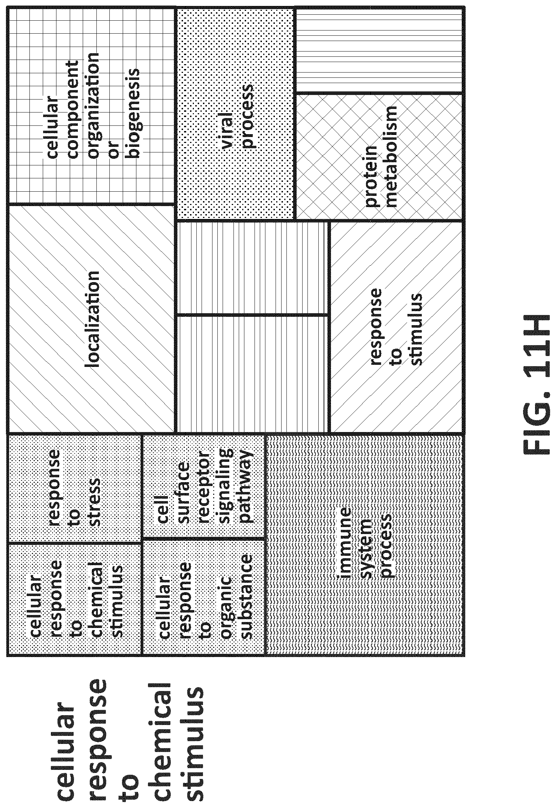

**Figure 11****.** Treemap diagram of biological processes related to isolated EVs of MSCs and MSCs-T-HIF^{c} using REVIGO after proteomic analysis. A) extracellular structure organization, B) vesicle mediated transport, C) anatomical structure morphogenesis and cell adhesion, D) wound healing, E) regulation of cellular component organization, F) immune effector process, G) secretion by cell, H) cellular response to chemical stimulus.

### Detailed description of the invention

Based on the existing needs and problems in the prior art, the present invention provides extracellular vesicles with improved therapeutic potency through genetic modification of parental MSCs.

The extracellular vesicles of the invention are secreted and isolated vesicles by genetically modified MSCs (MSCs-T-HIF), in which the overexpression of HIF-1α gives rise to a greater therapeutic potential and the overexpression of hTERT generates the unlimited and constant division of the cells, giving rise to a stable and homogeneous source of vesicles with improved therapeutic functions.

Therefore, in a first embodiment, the present invention refers to Extracellular Vesicles (EVs) isolated from mesenchymal stromal cells genetically modified to overexpress HIF-1α and hTERT (described herein as EV_{MSC-T-HIF}^{c} or EVs of the invention).

Parental MSCs can be isolated from different sources such as bone marrow, fat tissue, umbilical cord, peripheral blood or Warton gelly. Preferably, EVs of the present invention are isolated from human mesenchymal stromal cells derived from dental pulp.

In a second embodiment, the invention refers to a method for obtaining extracellular vesicles (EVs) isolated from mesenchymal stromal cells genetically modified to overexpress HIF-1α and hTERT (MSCs-T-HIF) (method of the invention). The method comprises the steps of:
a) Immortalizing a culture of MSCs by overexpressing the human telomerase enzyme (hTERT) by lentiviral vector, obtaining MSC-T,
b) Overexpressing HIF-1α in the immortalized MSCs-T, by lentiviral infection, obtaining MSCs-T-HIF and
c) Culturing the MSCs-T-HIF obtained in b) in a culture medium comprising a combination of the cytokines TNF-α, IFN-γ and IL-1β, during at least 48 hours, and
d) Isolating the EVs released by the MSCs-T-HIF to the culture medium.

In a third embodiment, the present invention refers to those extracellular vesicles obtained according to the method of the invention.

The EVs of the invention, which can be obtained by the method provided herein, show particular characteristics respect to extracellular vesicles isolated from unmodified MSCs, as described below.

For instance, the miRNA profile of the EVs of the invention are different compared to EVs secreted by unmodified MSCs.

In particular embodiments, the EVs show a miRNA profile with at least one of the following miRNAs upregulated in comparison to EVs isolated from unmodified MSCs:
hsa-miR-92b-3p, hsa-miR-186-5p, hsa-miR-4254, hsa-miR-6720-3p, hsa-miR-938, hsa-miR-138-1-3p, hsa-miR-222-3p, hsa-miR-5089-3p, hsa-miR-654-3p, hsa-miR-143-3p, hsa-miR-30a-5p, hsa-miR-7157-3p, hsa-miR-5585-5p, hsa-miR-590-3p, hsa-miR-12132, hsa-miR-16-5p, hsa-miR-22-3p, hsa-miR-150-5p, hsa-miR-199a-3p, hsa-miR-199a-5p, hsa-miR-199b-3p, hsa-miR-222-5p, hsa-miR-423-5p, hsa-miR-4732-3p and hsa-miR-4766-5p,
and/or at least one of the following miRNAs downregulated in comparison to EVs isolated from unmodified MSCs:
hsa-miR-6789-5p, hsa-miR-1539, hsa-miR-4686, hsa-miR-382-5p, hsa-miR-3916, hsa-miR-1909-5p, hsa-miR-103b, hsa-miR-486-5p, hsa-miR-4746-5p, hsa-miR-191-5p, hsa-miR-874-5p, hsa-miR-3685, hsa-miR-328-3p, hsa-miR-4725-5p, hsa-miR-6840-5p, hsa-miR-11181-3p, hsa-miR-877-3p, hsa-miR-301a-3p, hsa-miR-125a-3p, hsa-miR-181b-5p, hsa-miR-6843-3p, hsa-miR-6722-5p, hsa-miR-4727-3p, hsa-miR-3158-3p, hsa-miR-3158-5p, hsa-miR-6831-5p, hsa-miR-6510-3p, hsa-miR-3059-5p, hsa-miR-99a-5p, hsa-miR-4684-3p, hsa-miR-4740-3p, hsa-miR-3168, hsa-miR-5004-5p, hsa-miR-10401-5p, hsa-miR-7152-3p, hsa-miR-1272, hsa-miR-1295b-3p, hsa-miR-140-5p, hsa-miR-92a-3p, hsa-miR-127-3p, hsa-miR-4271, hsa-miR-487a-5p, hsa-miR-12131, hsa-miR-1255b-2-3p, hsa-miR-7113-3p, hsa-miR-5703, hsa-miR-6884-3p, hsa-miR-6070, hsa-miR-6816-3p, hsa-miR-181d-5p, hsa-miR-3678-3p, hsa-miR-6781-5p, hsa-miR-3617-3p, hsa-miR-933, hsa-miR-30c-2-3p, hsa-miR-543, hsa-miR-126-5p, hsa-miR-6507-5p, hsa-miR-4440, hsa-miR-10394-5p, hsa-miR-92b-5p, hsa-miR-370-5p, hsa-miR-5001-3p, hsa-miR-6737-3p, hsa-miR-6084, hsa-miR-34a-5p, hsa-miR-6835-5p, hsa-miR-4483, hsa-miR-8088, hsa-miR-6820-3p, hsa-miR-509-5p, hsa-miR-4282, hsa-miR-10b-5p, hsa-miR-454-3p, hsa-miR-378b, hsa-miR-1273h-5p, hsa-miR-1297, hsa-miR-3689a-3p, hsa-miR-4637, hsa-miR-493-5p, hsa-miR-6775-3p, hsa-miR-1258, hsa-miR-640, hsa-miR-1255b-5p, hsa-miR-548aw, hsa-miR-3126-3p and hsa-miR-758-3p.

Furthermore, the EVs of the present invention release PD-L1 in a higher concentration than EVs from unmodified MSCs. PD-1 is a protein on the surface of cells that has a role in regulating the immune system's response to the cells of the human body by down-regulating the immune system and promoting self-tolerance by suppressing T cell inflammatory activity. This prevents autoimmune diseases, but it can also prevent the immune system from killing cancer cells. In a particular embodiment, the EVs of the present invention release PD-L1 in an amount equal or higher than 1 pg/10¹¹ EVs.

Also, the protein profile of EVs_{MSC-T-HIF} is different compared to protein profile of EVs from unmodified MSCs (Table 1). Protein cargo involved in immune effector processes, immune system processes, secretion by cell, and response to stimulus are over-represented in primed EVs_{MSC-T-HIF} **(****Figure 11****).**

Genetic modifications introduced in parental MSCs, as explained below, produce extracellular vesicles with a high therapeutic potential. The EVs of the present invention unexpectedly show an increased immunosuppressive capacity, higher than EVs from wildtype parental cells.

Accordingly, the EVs of the invention can be used in the treatment of autoimmune diseases. Further, the EVs of the invention can be used in the treatment of ischemic diseases.

In a fourth embodiment, the present invention refers to a pharmaceutical composition comprising the EVs of the present invention.

The pharmaceutical composition is useful in cell therapy, in particular for the treatment of autoimmune diseases, such as graft versus host disease, COVID-19, sepsis, acute solid organ rejection and colitis and for use in the treatment of ischemic diseases, such as myocardial infarction, stroke and hind limb ischemia
The pharmaceutical compositions can be administered to the subject at a suitable dose. The dosage regimen depends upon many factors, including the patient's size, body surface area, age, sex, general health, other drugs being administered concurrently, time and route of administration. The composition of the invention is preferably administered to the subject intravenously or by local injection.

### Examples

### Materials and Methods

### Cell culture

Human dental pulp MSCs were cultured as previously described (Gandia, C. et al. Human Dental Pulp Stem Cells Improve Left Ventricular Function, Induce Angiogenesis, and Reduce Infarct Size in Rats with Acute Myocardial Infarction. Stem Cells 26, 638-645 (2008*).* Briefly, cells were cultured in Dulbecco's modified Eagle's medium (DMEM) low glucose (Sigma-Aldrich, Spain) supplemented with 10% heat-inactivated fetal bovine serum (FBS; Corning, Manassas, EEUU), 100 U/ml penicillin and 100 µg/ml streptomycin (P/S, Millipore). For EVs obtention, MSCs were cultured in extraction medium (EM) which was prepared by supplementing DMEM with 10% EVs depleted FBS and antibiotics. Depleted FBS was generated by ultracentrifugation of regular FBS and DMEM mixed 1:1 ratio and ultracentrifugated at 100,000 g for 16h. Buffy coat of healthy donors were obtained from Centro de Transfusion de la Comunidad Valencia, Spain after informed consent and peripheral blood mononuclear cells (PBMCs) were isolated by density gradient centrifugation with Histopaque (Sigma-Aldrich, St. Loius, MO). Isolated PBMCs were cultured in RPMI (Thermo Fisher Scientific) supplemented with 10% FBS, 2 mM glutamine, 100 U/ml penicillin and 100 µg/ml streptomycin (P/S, Millipore).

### MSC priming medium

Priming medium consisted of EM supplemented with IFN-γ (50ng/ml), TNF-α (10ng/ml) and IL-1β (10ng/ml).

### MSC differentiation

For osteogenic differentiation, cells were seeded at a density of 15.000 cells/cm² and cultured in osteogenic medium (OM) for 21 days renewing OM every 3 days. The osteogenic medium is composed by complete DMEM supplemented with 100 nM of Dexamethasone, 50 µM of Ascorbic Acid and 10 mM of Glycerol 2-phosphate (Sigma-Aldrich). Terminal differentiation was evaluated by Alizarin Red staining. For adipogenic differentiation, MSCs were seeded at a density of 25,000 cells/cm² and cultured in adipogenic medium for 21 days renewing AM every 3 days. AM was composed of complete DMEM supplemented with 1 µM dexamethasone, 200 µM indomethacin and 500 µM 3-isobutyl-1-methylxantine (Sigma-Aldrich). Terminal differentiation was monitored by observing lipid droplets inside cells.

### Lentiviral production and MSC transduction

Viral particles were produced in human embryonic kidney 293T cells (ATCC, www.atcc.org). Briefly, 293T cells were seeded in high-glucose DMEM containing 10% FBS. pMD2.G (Addgene, 12259), psPAX2 (Addgene, 12260), and the lentiviral vector carrying the transgene of interest were transfected into the packaging cell line by calcium phosphate precipitation. MSC transduction was carried out at a multiplicity of infection of 10 in order to achieve >95% infection. pLV-hTERT-IRES-hygro was obtained from Addgene (Addgene, 85140). The efficiency of transduction was evaluated by hygromycin selection test. pWPI-HIF-1α-GFP vector was previously described (Cerrada, I. et al. Hypoxia-Inducible Factor 1 Alpha Contributes to Cardiac Healing in Mesenchymal Stem Cells-Mediated Cardiac Repair. Stem Cells Dev. 22, 501-511 (2013*).* Transduction efficiency was evaluated quantifying GFP+ cells by flow cytometry. To assess the proliferative capacity of MSC transduced with hTERT or both hTERT and HIF-1α-GFP, MSC-T and MSC-T-HIF, respectively, parallel cultures from three different donors were submitted to 14 serial passages. Population doublings (PD) were calculated using the formula PD=[(log10(NH)-log10(N1)/log10(2) where NH is the cell harvest number and N1 the inoculum cell number (Kern S, Eichler H, Stoeve J, Klüter H, Bieback K. Comparative Analysis of Mesenchymal Stem Cells from Bone Marrow, Umbilical Cord Blood, or Adipose Tissue. Stem Cells [Internet]. Wiley; 2006 [cited 2020 Jul 20];24:1294-301. Available from: https://pubmed.ncbi.nlm.nih.gov/16410387/*). Cumulative population doublings (CPD) were calculated adding to each passage the PD of the previous passage).*

### Hygromycin selection test

Different MSC cell lines were seeded at 2.5x10⁴ cells/cm² density and incubated with 100 µg/ml of hygromycin for 48h. Cell viability was measured using MTT assay. Briefly, after incubation, cells washed and 0.5 mg/ml MTT solution added for 3 hours. Intracellular formazan was released with DMSO and absorbance measured by absorbance at 550 nm in a Halo Led 96 spectrophotometer (Dynamica Scientific).

### Telomerase activity

Telomerase activity was determined using a protocol described in Herbert et. Al (2006). Briefly, cell lysate was incubated with TS primer (SEQ ID NO 1, Sigma-Aldrich), ACX reverse primer (SEQ ID NO 2, Sigma-Aldrich), SYBR Green PCR Master Mix (Applied Biosystems) and EGTA (ethylene glycol bis (β-aminoethyl ether) -N, N, N', N'-N-tetraacetic acid) at 25°C for 20 minutes. Reaction product was quantified in a ViiA 7 Real-time PCR system. Relative telomerase activity (RTA) was extrapolated from a standard curve performed with HEK-293T cells.

### Senescence associated β-galactosidase assay

Senescence associated β-galactosidase assay was performed on MSCs seeded at low MSCs using β-Galactosidase Staining Kit (#9860, CST) following the manufacturer's instructions.

### EVs isolation and characterization

We used serial ultracentrifugation protocol described previously (*Dorronsoro A. et al, 2018*). Briefly, EVs containing supernatant was centrifuged at 2,000 g for 20 min (Eppendorf 5804 benchtop centrifuge, A-4-62 rotor), followed by centrifugation at 10,000 g for 70 min (Hitachi CP100NX centrifuge, Beckman Coulter 50.2 Ti rotor) and subsequently filtered manually through a 0.22 µm using a syringe. Afterwards, EVs were concentrated by two rounds of ultracentrifugation at 110,000 g for 120 min (Hitachi CP100NX centrifuge, Beckman Coulter 50.2 Ti rotor) with a washing step in between. When required, sample were passed through a 100 KDa AmiconTM filter (Merck Millipore) to remove the added cytokines and filtered through a 0.22 µm filter to maintain sterility. EVs protein concentration was determined with the Pierce BCA Protein Assay Kit (ThermoFisher Scientific) to ensure equal amounts of protein were added to the subsequent experiments. EVs were suspended in RIPA buffer [1% NP40, 0.5% deoxycholate, 0.1% sodium dodecyl sulfate in Tris-buffered saline (TBS), (Sigma-Aldrich) for western blotting and in phosphate buffered saline solution (PBS) for characterization and functional experiments. EVs size distribution and quantification of vesicles was analyzed by Nanoparticle tracking analysis (NTA) using a NanoSight NS3000 System (Malvern Instruments, UK). Electron microscopy was performed as previously described 23. Briefly, isolated EVs were diluted in PBS, loaded onto Formwar carbon-coated grids, contrasted with 2% uranyl acetate and finally examined with a FEI Tecnai G2 Spirit transmission electron microscope. Images were acquired using a Morada CCD Camera (Olympus Soft Image Solutions GmbH). Acetylcholinesterase assay was developed as previously described 24. Thirty microliters of each EVs preparation was added to individual wells of a 96-well flat-bottomed microplate. Then, 1.25 mM acetylthiocholine and 0.1 mM 5, 5'-di-thio-bis (North Rhine-Westphalia, Nümbrecht, Germany; http://sarstedt.com) (2-nitrobenzoic acid) were added to each well to a final volume of 300 µl and the change in absorbance at 412 nm was monitored every 10 minutes. Data are represented as Ac-CO activity after 30 minutes of incubation at 37°C.

### T cell proliferation assays

T cell proliferation assay was performed as previously described (Dorronsoro, A. et al. Human mesenchymal stromal cells modulate T-cell responses through TNF-alpha-mediated activation of NF-kappaB. Eur J Immunol 44, 480-488 (2014*).* Prior culturing, PBMCs were labeled with 5 µM carboxyfluoroscein succinimidyl ester (CFSE; ThermoFisher Scientific) following manufacturer instructions and activated with Dynabeads^{™} Human T-Activator CD3/CD28 (ThermoFisher Scientific). To evaluate EVs immunosuppressive potential, we added 15µg of different EVs extracts to 105 CFSE labelled PBMCs in 0.5 ml of medium. After 5 days of culture, proliferation of T cells was determined by flow cytometry measuring CFSE dilution. Analyses of flow cytometry data and Expansion Index (Lyons, A. B. Analysing cell division in vivo and in vitro using flow cytometric measurement of CFSE dye dilution. J. Immunol. Methods (2000). doi:10.1016/S0022-1759(00)00231-3), were performed using FlowJo software (Ashland, Oregon-based FlowJo LLC, BD). Percentage of immunosuppression was calculated normalizing data to a 0-100% scale by stablishing 0% immunosuppression for the expansion index of activated PBMCs not treated with EVs and 100% immunosuppression for none activated PBMCs' expansion index. CD3+ T cells and CD4+ and CD8+ positive subpopulations were identified by cells surface antigen staining.

### Immunofluorescence

To determine the intracellular localization of p65, cells were fixed and permeabilized with cold 70% Ethanol solution and the staining was performed using a goat anti-human p65 (Santa Cruz, sc-372) Ab, and a secondary antibody, goat anti-rabbit IgG (1:1,000; Alexa Fluor^{®} 568, Abcam). Nuclei were stained with DAPI. Quantification of fluorescence location was performed using ImageJ software (NIH).

### Real time quantitative PCR

RNA was extracted using TRIzol reagent (Invitrogen) and purified with the RNeasy Plus Mini Kit (Qiagen, Dusseldorf, Germany). cDNA was obtained by reverse transcription using M-MLV reverse transcriptase (Invitrogen). RT-qPCR was performed with SYBR^{™} Green PCR Master Mix (Applied Biosystems) and the following human-specific sense and antisense primers designed using: GAPDH SEQ ID NO 3 (F) and SEQ ID NO 4 (R); TERT SEQ ID NO 5 (F) and SEQ ID NO 6 (R); IDO SEQ ID NO 7 (F) and SEQ ID NO 8; COX2 SEQ ID NO 9 (F) and SEQ ID NO 10 (R); PD-L1 SEQ ID NO 11 (F) and SEQ ID NO 12 (R); IL6 SEQ ID NO 13 (F) and SEQ ID NO 14 (R); HIF-1α SEQ ID NO 15 (F) and SEQ ID NO 16 (R). The reaction was performed using a Viia 7 PCR System (Applied Biosystems).

### Western blot analysis

EVs or cells were lysed in 100 µL of RIPA buffer containing protease (Complete, Sigma-Aldrich) and phosphatase (PhosSTOP, Sigma-Aldrich) inhibitors. Equal amounts of protein samples were mixed with non-reducing Laemmli sample buffer (BioRad) and denatured at 96°C for 5 min. Proteins were separated on 10% SDS-polyacrylamide gels and transferred to polyvinylidene difluoride membranes (Immobilon-P; Millipore). Membranes were blocked with TBS containing 5% (w/v) non-fat dry milk powder with 0.1% Tween-20. Human primary antibodies used for WB: anti-Tubulin (dilution; Sigma-Aldrich; T5168), anti-hTERT (dilution; Rockland Immunochemicals; 600-401-252S), anti-HIF-1α (dilution; BD Biosciences; 610958), anti-IDO (dilution; Cell signal; #12006), anti-COX2 (dilution; Santa Cruz; H-3), anti-IL-6 (dilution; Santa Cruz; H-183), anti-HSP70 (dilution; Cell Signalling Technology; D69), anti-CD9 (dilution; Santa Cruz; C-4), anti-Tsg101 (dilution; Santa Cruz; C-2), anti-Calnexin (dilution; Santa Cruz, H-70) and anti-PD-L1 (dilution; AB Clonal; A11273). Secondary antibodies used: anti-IgG rabbit (dilution; Dako; P0448) and anti-IgG mouse (dilution; Sigma-Aldrich; A9044). Detection was carried out using peroxidase-conjugated secondary antibodies and with the ECL Plus Reagent (Amersham, GE Healthcare, Munich, Germany). Reaction was visualized using an Amershan Imager 600 (GE Healthcare) and quantified with ImageJ software (NIH).

### Flow cytometry

For flow cytometry analysis, EVs or cells were previously incubated with a blocking solution (PBS containing 1% NMS) for 10 min and then incubated with saturating amounts of fluorochrome-conjugated antibodies for 30 min at 4°C. Human antibodies used for Flow citometry: anti-CD14 (RPE, Dako; TUK4), anti-CD34 (APC, Dako; C7238), anti-CD45 (PECy5, BD Biosciences; HI-30), anti-CD73 (BV421, BD Biosciences; AD2), anti-CD90 (PE, Immunotech; F15-42-1-5), anti-CD105 (PE, Abcam; SN6), anti-CD3 (PerCP-Cy, BD Biosciences; SK7), anti-CD4(BV510, BD Biosciences; L200), anti-CD8 (PE-Cy7, BD Biosciences; RPA-T8) and anti-CD274 (APC, BD Biosciences; MIH1) at concentrations recommended by manufacturers and analyzed using a BD FACSCANTO II flow cytometer equipped with a Flowjo ^{®} software.

### PD-L1 measurement

Levels of PD-L1 in EVs were assayed by enzyme-linked immunosorbent assay (ELISA; R&D Systems) following the manufacturer's procedures.

### Delayed hypersensitivity type IV murine model

Adult male Balb/c mice (6 weeks old, 22-26 g) were purchased from Charles River Laboratories Inc., MA, USA, and housed in the IIS La Fe Animal housing facility under standard conditions. All animal procedures were approved by IIS La Fe ethical and animal care committees.

Mice were shaved and sensitized through abdominal topical application of 150 µl of 3% oxazolone (Santa Cruz) dissolved in acetone 5 days before the experiment. On day 5 post-sensitization, the mice were challenged by topical application of 20 µl of 1% of oxazolone dissolved in acetone on the inner and outer right ear surfaces. After 6h challenging, treatment of 15 µg of EVs were administered by a subcutaneous injection in the back of the ear. 50 µl of sterilized and filtered PBS was used as a vehicle. Control group was only punctured with vehicle. Ear thickness was measured pre-sensitization and 6h, 24, 48 and 72 h after challenge by digital micrometre. The degree of swelling was calculated as the thickness of the right ear (challenged ear) minus the baseline thickness of the left ear (unchallenged ear). The mice were sacrificed, and the ears were collected for ulterior morphometric analysis.

### Histology and Immunofluerescence

Ear samples were fixed with ethanol, embedded in paraffin, cut into 5 µm thick sections and stained with hematoxylin-eosin (Sigma-Aldrich). Slides were visualized using a Leica DMD108 Digital Microscope (Leica Microsystems). For immunofluorescence analysis, slides were blocked with 5% neonatal goat serum (NGS) and 0.1% Triton X-100 in PBS for 1h. Slides were incubated with anti-CD45 (dilution; BD Biosciences) overnight in a humidified chamber at 4°C and then slides were washed three times for 5 min each in PBS. Slides were incubated with anti-rat IgG-Alexa 555 secondary antibody for 1 h and then washed three times for 5 min in PBS. To detect the cell nucleus, slides were mounted using VECTASHIELD with DAPI (Vector Laboratories, Burlingame, CA). The sections were observed and visualized under fluorescent microscope Leica DM2500 (Leica Microsystems). Final image processing and quantification were performed by ImageJ software (NIH).

### Statistical analysis

Data were expressed as mean ± standard error of the mean. The Student's t test was used for paired samples for intragroup comparisons and for unpaired samples in the comparison between groups When the distribution was not normal the Mann-Whitney U test was used to compare two means. Analyses were conducted with GraphPad Prism 8 software. Differences were considered statistically significant at p < 0.05 with a 95% confidence interval.

### RESULTS

### Overexpression of hTERT in dental pulp derived MSCs generates a non-senescent and stable cell line.

Nowadays developing a therapy based on small EVs secreted by MSC faces several difficulties of which producing enough parental cells to get large amount of EVs is one of the most challenging ones. To address this issue, a dental pulp derived MSC line was immortalized by overexpressing human telomerase enzyme (MSC-T) using a lentiviral vector (Fig. 1A). As shown in figure 1, transgene integration (Fig. 1B, C, D) provided telomerase activity (Fig. 1E) to the MSC which lead to the extension of the proliferative capacity as determined by the assessment of population doubling (Fig. 1F) achieved without appearance of senescence as determined by β-Galactosidase activity (Fig. 1G). With this approach, the time in which an MSC cell line can be used as a stable EVs source was significantly extended.

### Priming MSC-T triggers expression of immunosuppressive molecules and increases EV secretion.

MSC in steady stage show low immunosuppressive capacity and is after stimulation with proinflammatory molecules that stromal cells turn on that capacity. Based on previous works, a conditioning medium composed by TNF-α, IFN-γ and IL-1β that mimics a proinflamatory environment was developed. As shown in figure 2, cytokine-activated MSC-T (MSC-Tc) induced expression of several genes related to the immunosuppressive capacity of MSC, such as IDO, COX2 and PD-L1 (Fig. 2 A). The results were corroborated by western blot analysis (Fig. 2B) and flow cytometry (Fig.2C) compared to non-conditioned MSC-T. At the same time, it was observed that MSC-Tc released a greater amount of EVs measured by bicinchoninic acid, acetylcholinesterase assays and NTA (Fig. 2D, E, F, respectively) without altering other phenotypic features such as morphology, size and presence of EVs markers (Figure 7).

### Conditioning MSC-T increases the immunosuppressive capacity of EVs.

Based on the higher expression of immunomodulatory molecules observed in MSC-Tc, the capacity of these cells to secrete more suppressive vesicles compare to non-conditioned cells was evaluated. Thus, the immunomodulation capacity of EVs secreted by MSC-T and MSC-Tc in a T cell proliferation assay was evaluated by measuring CFSE dilution in T cells stimulated with CD3/CD28 beads. As show in figure 3, MSC-Tc derived EVs (EV_{MSC-T}^{c}) show improved capacity to regulate T cell proliferation affecting both CD4 and CD8 cells (Fig. 3A). T cell proliferation is directly linked to the activation of T cells. Pursuing to better understand the mechanism behind this phenomenon the amount of different immunosuppressive molecules was measured in compared EVs and found that EV_{MSC-T}^{c} are enriched in IDO and PD-L1 (Fig. 3B, C, D). All these molecules are related to immunosuppressive capacity of MSC.

### HIF-1α overexpression in MSC-T increases immunomodulatory capacity of secreted EVs.

In order to test if immunomodulatory benefits trigger by overexpression of HIF-1α in MSCs were conserved by secreted EVs, HIF-1α were transduced in the MSC-T line using lentivirus (MSC-T-HIF). Transduction efficiency was measured by flow cytometry detecting GFP in MSC-T cells and overexpression of the transgene quantified by WB and qPCR. MSC-T-HIF showed similar features to MSC-T in terms of immortalization and other MSC characteristic markers (figures 8, 9).

NF-kB pathway is a key regulator of immunosuppressive capacity of MSC. It has been described that activating NF-κB in MSC is an essential step to trigger the capacity of these cells to avoid T cell proliferation (Dorronsoro et al, 2014). Thus, NF-κB pathway activity was measured by immunolocalization of nuclear translocation of p65 and by measuring expression of IL-6 after conditioning with cytokine cocktail both MSC-T and MSC-T-HIF. In this manner, overexpressing HIF-1α triggers stronger response of MSCs to the conditioning cocktail were observed (Fig. 4 A). Accordingly, conditioned MSC-T-HIF (MSC-T-HIFc) express higher levels of immunosuppressive molecules, such IDO, COX2 and PD-L1 (Fig. 4B, C).

In terms of EVs secretion, it was observed that MSC-T-HIFc secreted higher amount of EVs compare to MSC-Tc (Fig. 5A, B, C). Next, it was measured how MSC-T-HIFc derived EVs (EV_{MSC-T-HIF}^{c}) performed in a T proliferation assay. As shown in figure 5, it was observed that EV_{MSC-T-HIF}^{c} have higher capacity to suppress T cell proliferation than EV_{MSC-T}^{c}, affecting both CD4 and CD8 subpopulations (Fig. 5D).

Following, it was studied which of the immunosuppressive molecules were enriched in EVs and could be related to the immunomodulation observed. As shown in figure 5D, EV_{MSC-T-HIF}^{c} show higher level of PD-L1 and IDO and (Fig. 5E). PD-1 is a protein on the urface of cells that has a role in regulating the immune system's response to the cells of the human body by down-regulating the immune system and promoting self-tolerance by suppressing T cell inflammatory activity. This prevents autoimmune diseases, but it can also prevent the immune system from killing cancer cells. The expression of PDL1 in the surface of EVs was further corroborated by flow cytometry and ELISA (Fig 5F, G).

### HIF overexpression and priming medium conditions causes alterations in the cargo of proteins and miRNAs of EVs

To functionally characterize the protein content of primed EVS_{MSC-T-HIF} compared non-primed EVS_{MSC}, LC-MS/MS was used **(table 1)**. Protein contaminants from the cell culture medium (serum albumin and keratin) were removed from the analysis. An equivalent amount of total protein was used (30 µg). We further mapped the biological processes and metabolic pathways represented by EVs proteins. The most significant biological processes identified in both conditions were visualized in a Treemap diagram using REVIGO **(****figure 11****)**. Non-primed EVs_{MSC} contained protein cargo involved in ECM organization, vesicle-mediated transport, wound healing and cell adhesion, whereas additional biological processes were over-represented in primed EVs_{MSC-T-HIF}, such as immune effector process, immune system process, secretion by cell, and response to stimulus.

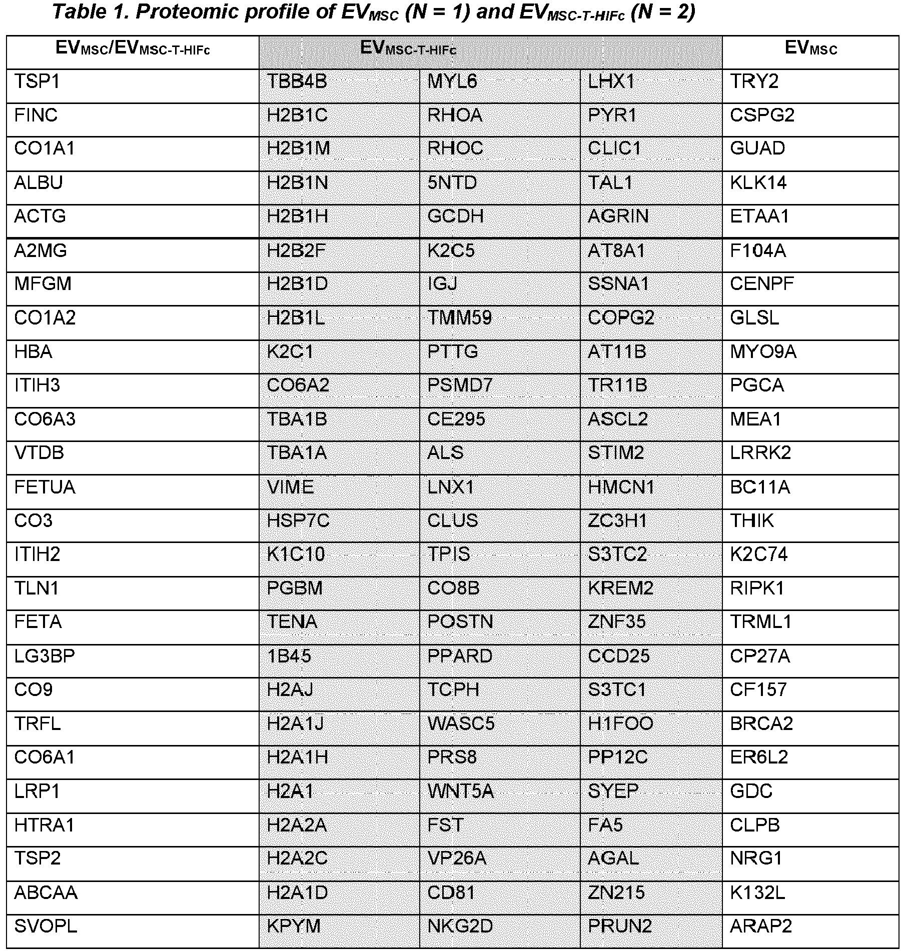

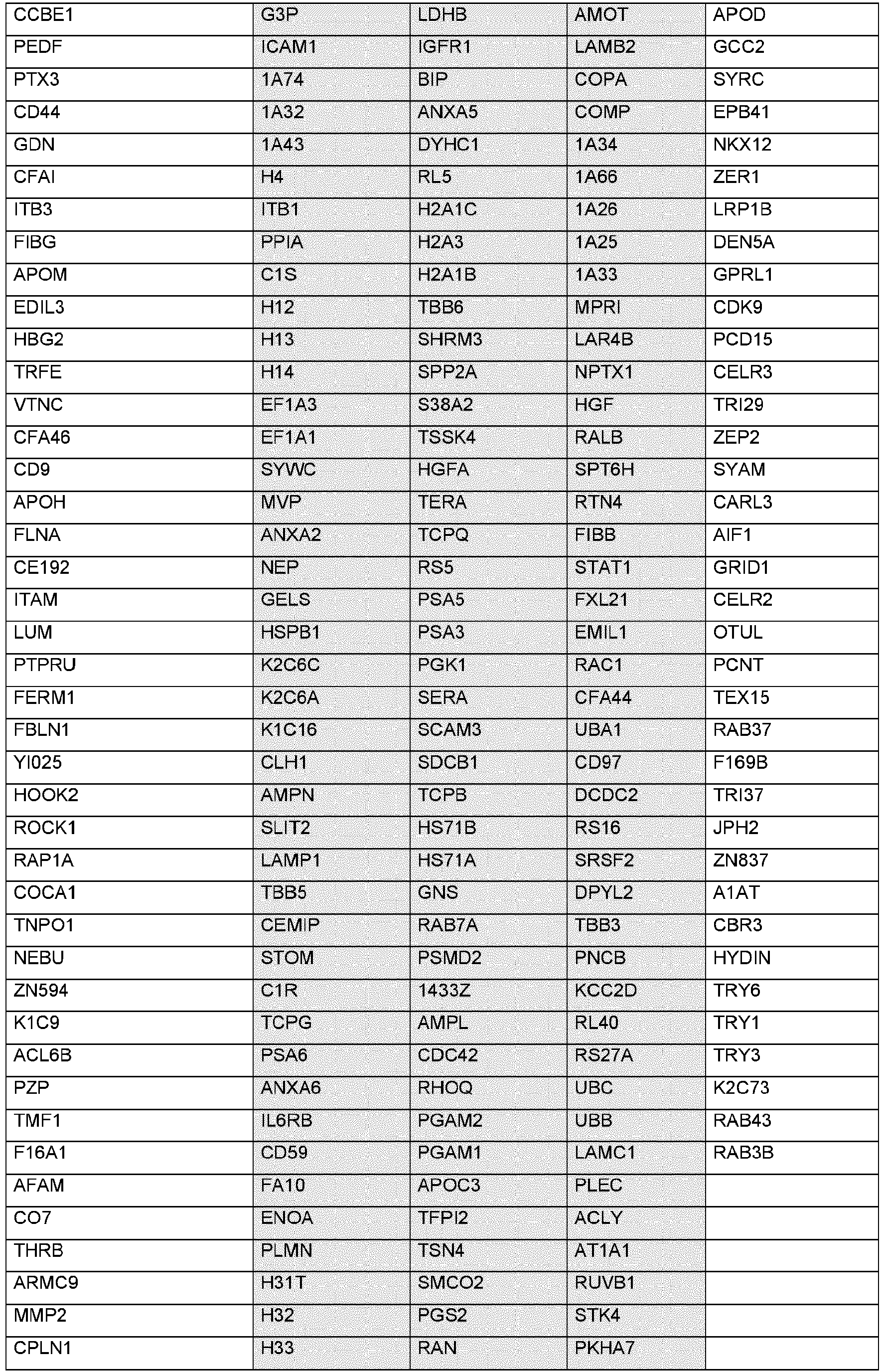

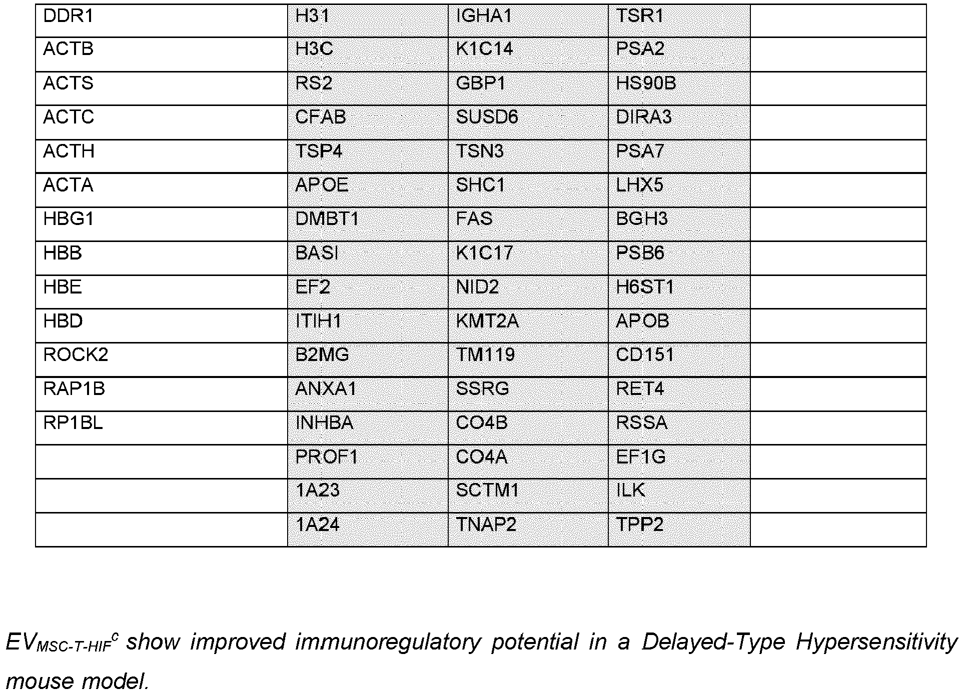

In vitro assays used in immunology research are useful to study the role of treatments over each type of blood cells; however in vivo immune reactions are coordinated multicellular processes that require in vivo models to unveil their complexity. Thus, the immunosuppressive capacity of EVs of the present invention in a Delayed-Type Hypersensitivity mouse model (DTH) was tested. DTH is a useful approach for evaluating immune response mediated by T cells and monocytes/macrophages (Allen, I. C. Delayed-Type Hypersensitivity Models in Mice. in Methods in Molecular Biology 1031, 101-107 (2013*).* The inflammatory response is characterized by swelling of ear due to rapid recruitment of immune cells to the ear in response to oxazolone. Severity of the reaction is evaluated by measuring ear thickness and analysing tissue integrity and infiltration by H&E staining and immunofluorescence. Using this model, it was observed that both EV_{MSC-T}^{c} and EV_{MSC-T-HIF}^{c} reduced the inflammatory reaction in the ear (Fig. 6 A). Moreover, it was observed that EV_{MSC-T-HFc} are significantly more effective. As show in figure 6B, EV_{MSC-T-HIFc} reduce swelling to 38.64±2.88% 48 hours after challenging and to 40.76±7.18% 72 hours after. Histological analysis of challenged ear showed that DTH reaction was associated with vascular enlargement and orthokeratotic hyperkeratosis (Fig. 6 C). In addition, a massive CD45+ cell infiltration in the challenged ear was observed (Fig. 6 D). These alterations were dramatically reduced in mice treated with a single dose of EVs. In conclusion, EV_{MSC-T-HIFc} were significantly more effective than EV_{MSC-Tc} (Fig. 6C, D).

The results indicated that, after EV_{MSC-T-HIF}^{c} uptake, immune cells display better immunosuppression features. EV_{MSC-T-HIF}^{c} reduced proliferation of activated T cells more effectively than EVs from wildtype parental cells. Additionally, a delayed hypersensitivity type IV (DTH) mouse model was used to validate *in vitro* results. EVs_{MSC-T-HIF}^{c} subcutaneous injection significantly reduced the swelling of mouse ear, declined immune cell infiltration and improved tissue integrity.

## Claims

1. Extracellular vesicles (EVs) isolated from mesenchymal stromal cells genetically modified to overexpress HIF-1α and hTERT (MSCs-T-HIF).

2. Method for obtaining extracellular vesicles (EVs) isolated from mesenchymal stromal cells genetically modified to overexpress HIF-1α and hTERT (MSCs-T-HIF), according to claim 1, comprising the steps of:
a. Immortalizing a culture of MSCs by overexpressing the human telomerase enzyme (hTERT) by lentiviral vector, obtaining MSC-T,
b. Overexpressing HIF-1α in the immortalized MSCs-T, by lentiviral infection, obtaining MSCs-T-HIF and
c. Culturing the MSCs-T-HIF in a culture medium comprising a combination of the cytokines TNF-α, IFN-γ and IL-1β, during at least 48 hours, and
d. Isolating the EVs released by the MSCs-T-HIF to the culture medium.

3. Extracellular vesicles obtained according to the method of claim 2.

4. EVs according to claim 1 or 3 wherein said EVs release PD-L1 in an amount equal or higher than 1 pg/10¹¹ EVs.

5. EVs according to claim 1, 3 or 4, wherein said EVs show a modified miRNA profile, with respect to that of EVs obtained from unmodified MSC cells, with at least one of the following miRNAs upregulated:
hsa-miR-92b-3p, hsa-miR-186-5p, hsa-miR-4254, hsa-miR-6720-3p, hsa-miR-938, hsa-miR-138-1-3p, hsa-miR-222-3p, hsa-miR-5089-3p, hsa-miR-654-3p, hsa-miR-143-3p, hsa-miR-30a-5p, hsa-miR-7157-3p, hsa-miR-5585-5p, hsa-miR-590-3p, hsa-miR-12132, hsa-miR-16-5p, hsa-miR-22-3p, hsa-miR-150-5p, hsa-miR-199a-3p, hsa-miR-199a-5p, hsa-miR-199b-3p, hsa-miR-222-5p, hsa-miR-423-5p, hsa-miR-4732-3p and hsa-miR-4766-5p,
and/or at least one of the following miRNAs downregulated:
hsa-miR-6789-5p, hsa-miR-1539, hsa-miR-4686, hsa-miR-382-5p, hsa-miR-3916, hsa-miR-1909-5p, hsa-miR-103b, hsa-miR-486-5p, hsa-miR-4746-5p, hsa-miR-191-5p, hsa-miR-874-5p, hsa-miR-3685, hsa-miR-328-3p, hsa-miR-4725-5p, hsa-miR-6840-5p, hsa-miR-11181-3p, hsa-miR-877-3p, hsa-miR-301a-3p, hsa-miR-125a-3p, hsa-miR-181b-5p, hsa-miR-6843-3p, hsa-miR-6722-5p, hsa-miR-4727-3p, hsa-miR-3158-3p, hsa-miR-3158-5p, hsa-miR-6831-5p, hsa-miR-6510-3p, hsa-miR-3059-5p, hsa-miR-99a-5p, hsa-miR-4684-3p, hsa-miR-4740-3p, hsa-miR-3168, hsa-miR-5004-5p, hsa-miR-10401-5p, hsa-miR-7152-3p, hsa-miR-1272, hsa-miR-1295b-3p, hsa-miR-140-5p, hsa-miR-92a-3p, hsa-miR-127-3p, hsa-miR-4271, hsa-miR-487a-5p, hsa-miR-12131, hsa-miR-1255b-2-3p, hsa-miR-7113-3p, hsa-miR-5703, hsa-miR-6884-3p, hsa-miR-6070, hsa-miR-6816-3p, hsa-miR-181d-5p, hsa-miR-3678-3p, hsa-miR-6781-5p, hsa-miR-3617-3p, hsa-miR-933, hsa-miR-30c-2-3p, hsa-miR-543, hsa-miR-126-5p, hsa-miR-6507-5p, hsa-miR-4440, hsa-miR-10394-5p, hsa-miR-92b-5p, hsa-miR-370-5p, hsa-miR-5001-3p, hsa-miR-6737-3p, hsa-miR-6084, hsa-miR-34a-5p, hsa-miR-6835-5p, hsa-miR-4483, hsa-miR-8088, hsa-miR-6820-3p, hsa-miR-509-5p, hsa-miR-4282, hsa-miR-10b-5p, hsa-miR-454-3p, hsa-miR-378b, hsa-miR-1273h-5p, hsa-miR-1297, hsa-miR-3689a-3p, hsa-miR-4637, hsa-miR-493-5p, hsa-miR-6775-3p, hsa-miR-1258, hsa-miR-640, hsa-miR-1255b-5p, hsa-miR-548aw, hsa-miR-3126-3p and hsa-miR-758-3p,

6. Extracellular vesicles, according to any of claims 1 and 3-5 wherein the mesenchymal stromal cells are human mesenchymal stromal cells derived from dental pulp.

7. A pharmaceutical composition comprising EVs according to any of claims 1 and 3-6.

8. The pharmaceutical composition, according to claim 7 for use in cell therapy.

9. The pharmaceutical composition, according to claims 7 or 8 for use in the treatment of autoimmune diseases.

10. The pharmaceutical composition, according to claim 9, wherein the autoimmune disease is selected from graft versus host disease, COVID-19, sepsis, arthritis, acute solid organ rejection and colitis.

11. The pharmaceutical composition, according to claim 7 or 8 for use in the treatment of ischemic diseases.
